(19) Europäisches Patentamt
European Patent Office
Office européen des brevets

(11) **EP 1 405 071 B1**

(12) **EUROPÄISCHE PATENTSCHRIFT**

(45) Veröffentlichungstag und Bekanntmachung des Hinweises auf die Patenterteilung:
**29.10.2008   Patentblatt 2008/44**

(51) Int Cl.:
*G01N 33/487* (2006.01)   *H03F 3/45* (2006.01)

(21) Anmeldenummer: 02754342.0

(22) Anmeldetag: **10.07.2002**

(86) Internationale Anmeldenummer:
**PCT/DE2002/002526**

(87) Internationale Veröffentlichungsnummer:
**WO 2003/006981 (23.01.2003 Gazette 2003/04)**

(54) **MESSZELLE ZUR AUFNAHME DES ELEKTRISCHEN POTENTIALS EINER PROBE**

MEASURING CELL AND MEASURING FIELD COMPRISING MEASURING CELLS OF THIS TYPE, USE OF A MEASURING CELL AND USE OF A MEASURING FIELD

CELLULE DE MESURE ET CHAMP DE MESURE POURVU DE TELLES CELLULES DE MESURE ; UTILISATION D'UNE CELLULE DE MESURE ET UTILISATION D'UN CHAMP DE MESURE

(84) Benannte Vertragsstaaten:
**DE FR GB IT**

(30) Priorität: **10.07.2001   DE 10133363**

(43) Veröffentlichungstag der Anmeldung:
**07.04.2004   Patentblatt 2004/15**

(73) Patentinhaber: **Infineon Technologies AG 81669 München (DE)**

(72) Erfinder:
• **BREDERLOW, Ralf**
  **81737 München (DE)**
• **EVERSMANN, Björn-Oliver**
  **80336 München (DE)**
• **KOREN, Ivo**
  **81737 München (DE)**
• **PAULUS, Christian**
  **82362 Weilheim (DE)**
• **THEWES, Roland**
  **82194 Gröbenzell (DE)**

(74) Vertreter: **Viering, Jentschura & Partner Postfach 22 14 43 80504 München (DE)**

(56) Entgegenhaltungen:
**DE-C- 10 001 124**

• **BAUMANN W H ET AL: "Microelectronic sensor system for microphysiological application on living cells" SENSORS AND ACTUATORS B, ELSEVIER SEQUOIA S.A., LAUSANNE, CH, Bd. 55, Nr. 1, 25. April 1999 (1999-04-25), Seiten 77-89, XP004175066 ISSN: 0925-4005**
• **SMITH R ET AL: "Electrostatically protected ion sensitive field effect transistors" SENSORS AND ACTUATORS, FEB. 1984, SWITZERLAND, Bd. 5, Nr. 2, Seiten 127-136, XP008016313 ISSN: 0250-6874**
• **KEIJI TSUKADA ET AL: "ISFET INCORPORATED IN A DIFFERENTIAL AMPLIFIER" ELECTRONICS & COMMUNICATIONS IN JAPAN, PART II - ELECTRONICS, SCRIPTA TECHNICA. NEW YORK, US, Bd. 71, Nr. 3, 1. März 1988 (1988-03-01), Seiten 82-87, XP000035612 ISSN: 8756-663X**
• **TAKAHASHI K ET AL: "BI-MOSFET AMPLIFIER FOR INTEGRATION WITH MULTIMICROELECTRODE ARRAY FOR EXTRACELLULAR NEURONAL RECORDING" IEICE TRANSACTIONS ON FUNDAMENTALS OF ELECTRONICS, COMMUNICATIONS AND COMPUTER SCIENCES, INSTITUTE OF ELECTRONICS INFORMATION AND COMM. ENG. TOKYO, JP, Bd. E77-A, Nr. 2, 1. Februar 1994 (1994-02-01), Seiten 388-393, XP000447727 ISSN: 0916-8508 in der Anmeldung erwähnt**

- **STETT A ET AL: "TWO-WAY SILICON-NEURON INTERFACE BY ELECTRICAL INDUCTION" PHYSICAL REVIEW E. STATISTICAL PHYSICS, PLASMAS, FLUIDS, AND RELATED INTERDISCIPLINARY TOPICS, AMERICAN INSTITUTE OF PHYSICS, NEW YORK, NY, US, Bd. 55, Nr. 2, Februar 1997 (1997-02), Seiten 1779-1782, XP000913528 ISSN: 1063-651X in der Anmeldung erwähnt**

**EP 1 405 071 B1**

**Beschreibung**

In der Analytik beziehungsweise der Sensorik werden potentiometrische Messungen vorgenommen, d.h. es werden elektrische Potentiale ohne Einprägung eines Stromes in das Messobjekt gemessen. Diese elektrischen Potentiale stellen die zu charakterisierende Eigenschaft des Analyten dar oder werden aus der zu charakterisierenden Eigenschaft eines Analyten mittels eines sog. Transducers generiert. Ein Sensor dafür wandelt diese elektrischen Potentiale in elektrische Signale, beispielsweise Spannungssignale, die im folgenden ausgewertet werden. Zum Beispiel werden in der Grundlagenforschung der Medizin bei der potentiometrischen Messung extrazellulärer neuronaler Signale Elektroden verwendet.

Gemäß [1] ist zur Messung von elektrischen Potentialen von Neuronen ein Elektrodenbaustein vorgesehen. Der aus Halbleitermaterial hergestellte Elektrodenbaustein enthält eine spitz zulaufende Mikroelektrode und an ihrem breiten Ende ein rechteckiges Substrat mit einem Verstärker darauf.

Problem bei dem Elektrodenbaustein nach [1] ist, dass keine Neuronenverbände ortsaufgelöst simultan gemessen werden können, da durch die spitz zulaufende Mikroelektrode nur punktuelle Messungen vorgenommen werden können. Aufgrunddessen ist bei dem vorliegenden Elektrodenbaustein auch die Dimensionierung des Verstärkers von untergeordneter Bedeutung, da sich keinerlei Bauraumzwänge ergeben.

Aus [2] ist ein als Sensor verwendeter Feldeffekttransistor zum elektronischen Auslesen neuronaler Signale von lebenden Zellen bekannt. Eine lebende Nervenzelle liegt dabei auf dem isolierten, offenen Gate des Feldeffekttransistors auf. Mit diesem Sensor können neuronale Signale der Zelle, die sich in Form von Potentialänderungen an der Zellwand äußern, aufgenommen werden, da diese Potentialänderungen den Kanalstrom des Transistors beziehungsweise die Dichte der im Kanalbereich zwischen Drain und Source vorhandenen Ladungsträger steuern beziehungsweise modulieren.

In [3] wurde die aus [2] bekannte Anordnung dahingehend erweitert, dass mittels bidirektionaler Verbindung das Potential einer Nervenzelle durch Anlegen eines elektrischen Signals an die Anordnung verändert werden kann. Dazu ist neben der FET-Struktur isoliert von dieser eine Steuerelektrode angeordnet, die durch Anlegen eines entsprechenden elektrischen Signals die Nervenzelle zu einer Änderung ihres elektrischen Potentials veranlasst.

[4] zeigt die zuvor beschriebenen Anordnungen in der Anwendung bei der Ermittlung von Potentialen von Nervenzellen einer Ratte.

Bei einer an den oben beschriebenen Sensor angeschlossenen externen Signalverstärkung ist aufgrund der kleinen Signalamplituden eine schlechte Signalübertragung, ein schlechter Signal-zu-Rausch-Abstand, sowie eine höhere Empfindlichkeit gegenüber Störeinflüssen (z.B. Streufeldern) und parasitäre Effekte von Nachteil.

Aus [5] ist ein Feld aus ionensensitiven, auf einem GaAs-Substrat aufgebauten Feldeffekttransistoren bekannt, die in Reihen und Spalten angeordnet sind. Um die einzelnen Transistoren möglichst eng benachbart anordnen zu können, wurden die Gastes einer in einer gemeinsamen Spalte angeordneten FETs zu einem Back-Gate miteinander verbunden, wie auch die Kanäle von in einer Reihe liegenden FETs. Durch sequentiellen Abgriff von Spalten und Reihen kann der Ort einer Potentialänderung innerhalb des Messfeldes ermittelt werden.

Nachteilig an der Anordnung nach [5] ist, dass der Strompfad über eine Reihenschaltung von Sensoren fließen muss. Damit ist die Realisierung großer Felder / Arrays nicht möglich, da der Serienwiderstand bezüglich des ausgewählten Sensors zu einer Abschwächung des Signals gemäß einem Spannungsteiler führt.

Des weiteren ist ein sehr aufwendiger Herstellungsprozess notwendig, um ein Back-Gate zu realisieren. Ferner wird eine spezielle GaAs-Technologie verwendet, wobei das verwendete Material Arsen stark toxisch ist und der Sensor somit nur für einen geringen Anteil möglicher Anwendungen geeignet ist.

[6] zeigt ein Feld von ionensensitiven Feldeffekttransistoren auf einem gemeinsamen Substrat. Dabei dient das Feld nicht dazu, dass einzelne Transistoren lokale Impedanzen in ihrer Umgebung feststellen können, die sich von der auf den benachbarten Transistor einwirkenden Impedanz unterscheiden. Eine größtmögliche Auflösung des Feldes ist also gar nicht erwünscht. Statt dessen soll gerade die Messprobe über das Feld hinweg konstante Eigenschaften aufweisen, denn das Feld dient als Analog-Digital-Sensor: Dabei ist jeder als Feldeffekttransistor ausgebildeter Sensorzelle ein Schwellwert zugeordnet. Die Schwellwerte unterscheiden sich von Sensorzelle zu Sensorzelle. Eine Vergleichsschaltung überprüft, ob die Messgröße den jeweiligen Schwellwert überschreitet. Damit entsteht bezüglich einer das Feld überdeckenden Probe ein Feld aus binären Werten, das einen bestimmten Messwert wiedergibt. Ausleseschaltungen sind auf dem Substrat integriert.

[7] zeigt eine weiterentwickelte Anordnung nach [6]. Dabei ist jeder Sensorreihe nur ein Vergleicher zugeordnet, dessen Schwellwerte veränderbar sind, sodass die einzelnen Sensoren sequentiell ausgelesen werden.

[8] zeigt diesbezüglich weitere Mechanismen zur Schwellwertevaluierung.

Bei den zuletzt beschriebenen Anordnungen werden die so in den einzelnen Sensoren erzielten Ausgangswerte aufsummiert und gemittelt. Dieser Mittelwert hängt dann linear von der Messgröße ab.

Damit sind allerdings nur relativ langsame Messungen ohne Ortsauflösung möglich, da einerseits das gesamte Feld von der Probe überdeckt sein muss, damit alle Sensoren die gleiche Größe aufnehmen und mit den zugeordneten Schwellwerten vergleichen können. Zum anderen sind die Auslesemechanismen aufwendig und langsam, sodass die Anordnung nur für statische Messungen geeignet ist.

Für die ortsaufgelöste Messung von etwa sehr kleinen extrazellulären Signalen einzelner Neuronen innerhalb eines Verbandes von Neuronen ist dagegen eine sehr hohe Packungsdichte von Sensoren notwendig.

Um die Signale einzelner Neuronen messen zu können, müssen die Ausgangssignale der Sensoren des Feldes - im folgenden auch Array genannt - einzeln auslesbar sein. Der Abstand benachbarter Messzellen - im folgenden auch Pixel genannt -, die jeweils die Sensorschaltungen enthalten, muss kleiner als die Neuronen selbst sein. Für die Herstellung von Sensoren stellen aber zum einen durch den Herstellungsprozess bedingte minimale Strukturgrößen eine Rahmenbedingung dar. Zum anderen nehmen statistische Schwankungen der Parameter und der Rausch-Anteile der Sensorsignale mit abnehmender Fläche der Bauelemente stark zu.

Die Bauelemente der Sensorschaltungen müssen deshalb in vielen Anwendungen größer als minimal dimensioniert werden, um ein akzeptables Signal-zu-Rausch-Verhältnis zu garantieren. Damit müssen Pixelschaltungen mit einer möglichst geringen Anzahl an Bauelementen realisiert werden, um einen kleinen Pitch und gute Signal-zu-Rausch-Verhältnisse zu erreichen. Des weiteren müssen auch Stabilität, hohe Empfindlichkeit und Robustheit der Anordnung sichergestellt sein.

Ferner ist in [10] ein kapazitiver Abstands-Sensor zum Erfassen eines Fingerabdruck-Bildes beschrieben. Der Sensor weist ein kapazitives Element mit einer ersten und einer zweiten Kondensatorplatte auf, welche nebeneinander angeordnet sind und deren Abstand zueinander gemessen wird. Der Sensor weist ferner einen invertierenden Operationsverstärker auf, in dessen Rückkopplungszweig die kapazitiven Elemente geschaltet sind.

[11] beschreibt einen Messschaltkreis mit einem Biosensor, bei dem ionensensitive Feldeffekttransistoren eingesetzt werden.

[12] offenbart ein gemultiplextes aktives biologisches Elektrodenarray.

In [13] sind Grundschaltungen mit Feldeffekttransistoren beschrieben.

[14] beschreibt einen Sensor-Chip mit einem Ionensensitiven Feldeffekttransistor (ISFET) und einem mit diesem zu einem Differenzverstärker verschalteten MOS-Feldeffekttransistor, wobei die Gate-Isolation des ISFET gebildet wird von einer Doppelschicht aus $SI_3N_4$ und $SiO_2$. Die $SI_3N_4$-Teilschicht dient zum Erfassen des pH-Wertes einer auf diese aufgebrachten Flüssigkeit.

Der Erfindung liegt das Problem zugrunde, eine Messzelle, ein Messfeld mit Messzellen sowie ein Verfahren zum Einstellen eines Arbeitspunktes einer Verstärkerschaltung in einer Messzelle zu schaffen, die den gestellten Anforderungen genügen und robust und universell einsetzbar sind.

Das Problem wird gelöst durch eine Messzelle, ein Messfeld sowie ein Verfahren zum Einstellen eines Arbeitspunktes einer Verstärkerschaltung in einer Messzelle mit den Merkmalen gemäß den unabhängigen Patentansprüchen. Bevorzugte Ausgestaltungen der Erfindung ergeben sich aus den abhängigen Ansprüchen.

Die in ein Substrat integrierte Messzelle enthält einen Sensor, auch Wandlerelement genannt. Das Wandlerelement setzt eine Meßgröße in ein elektrisches Potentialum. Darunter fällt auch das bloße Übermitteln eines elektrischen Potentials.

Anschaulich kann die Erfindung darin gesehen werden, dass in jeweils einer Messzelle eine einstufige oder mehrstufige Verstärkerschaltung vorgesehen ist, deren Arbeitspunkt mittels eines Lastelements als Rückkopplungselement einstellbar ist. Damit wird es ermöglicht, mittels des Lastelements als Rückkopplungselement die Parameterschwankungen der Verstärkerschaltung zu kompensieren.

Ausführungsbeispiele der Erfindung sind in den Figuren dargestellt und werden im Weiteren näher erläutert.

Es zeigen

Figur 1; ein erstes elektrisches Schaltbild einer erfindungsgemäßen Messzelle;

Figur 2; ein zweites elektrisches Schaltbild einer erfindungsgemäßen Messzelle;

Figur 3; ein drittes elektrisches Schaltbild einer erfindungsgemäßen Messzelle;

Figur 4; ein viertes elektrisches Schaltbild einer erfindungsgemäßen Messzelle, vorgesehen zur Anordnung in einem Messfeld;

Figur 5; ein fünftes elektrisches Schaltbild einer erfindungsgemäßen Messzelle, vorgesehen zur Anordnung in einem Messfeld;

Figur 6; ein sechstes elektrisches Schaltbild einer erfindungsgemäßen Messzelle, vorgesehen zur Anordnung in einem Messfeld;

Figur 7;   ein siebtes elektrisches Schaltbild einer erfindungsgemäßen Messzelle, vorgesehen zur Anordnung in einem Messfeld;

Figur 8;   ein achtes elektrisches Schaltbild einer erfindungsgemäßen Messzelle, vorgesehen zur Anordnung in einem Messfeld;

Figur 9;   ein neuntes elektrisches Schaltbild einer erfindungsgemäßen Messzelle, vorgesehen zur Anordnung in einem Messfeld;

Figur 10;   Ausführungsbeispiele eines Transistors der auf dem Substrat angeordneten Verstärkerschaltung;

Figur 11;   Ausführungsbeispiele eines Lastelements der auf dem Substrat angeordneten Verstärkerschaltung;

Figur 12;   ein erstes elektrisches Schaltbild eines erfindungsgemäßen Messfeldes;

Figur 13;   ein zweites elektrisches Schaltbild eines erfindungsgemäßen Messfeldes;

Figur 14;   ein drittes elektrisches Schaltbild eines erfindungsgemäßen Messfeldes;

Figur 15;   ein viertes elektrisches Schaltbild eines erfindungsgemäßen Messfeldes;

Figur 16;   ein fünftes elektrisches Schaltbild eines erfindungsgemäßen Messfeldes;

Figur 17;   ein sechstes elektrisches Schaltbild eines erfindungsgemäßen Messfeldes;

Figur 18;   ein erstes erfindungsgemäßen Messfeldes in Draufsicht und in zwei Schnittansichten;

Figur 19;   ein zweites erfindungsgemäßen Messfeldes in Draufsicht und in zwei Schnittansichten.

Gleiche Elemente und Größen in den Figuren sind mit den gleichen Bezugszeichen versehen.

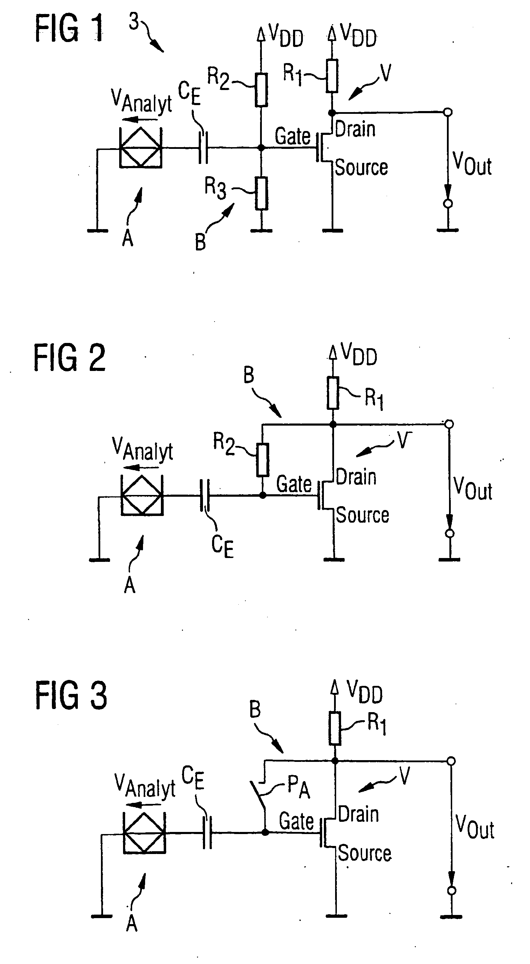

Figur 1 zeigt ein elektrisches Schaltbild einer erfindungsgemäßen Messzelle 3 mit einem äquivalenten Ersatzschaltbild eines Sensors A zum Wandeln eines Potentials, vorzugsweise des Potentials eines Neurons, in ein elektrisches Sensorsignal $V_{Analyt}$, vorzugsweise also ein Spannungssignal. Dabei ist der Sensor A als Feldeffekttransistor ausgebildet mit Gate, Source und Drain.

Die Probe ist im Sensorbetrieb über der Gateschicht angeordnet, liegt vorzugsweise auf einer auf der Gateschicht aufgebauten Isolatorschicht auf und steuert mit ihrem elektrischen Potential einen Kanalstrom des Feldeffekttransistors zwischen Source und Drain. Die zu detektierende Änderung des Potentials bzw. der Eigenschaft einer Probe / eines Analyten wird also über eine Änderung der Spannung $V_{Analyt}$ an dem Sensor A modelliert.

Der Sensor A ist einerseits mit Masse verbunden, andererseits über eine gleichspannungsentkoppelnde Kapazität $C_E$, der Kapazität der Elektrode, mit einer Verstärkerschaltung B mit einem einstufigem Verstärker. Es wird CMOS-Technologie verwendet.

Sensor A und Verstärkerschaltung B sind auf einem gemeinsamen Substrat integriert angeordnet.

Die als Vorverstärker betriebene Verstärkerschaltung enthält einen in Source-Schaltung betriebenen n-Typ-MOS-Feldeffekttransistor V mit Gate, Source und Drain. Über die als Widerstände ausgebildeten Lastelemente R1 bis R3 wird der Arbeitspunkt des Transistors V eingestellt. Der Widerstand $R_1$ verbindet Drain mit einem ersten Versorgungspotential $V_{DD}$. Der Arbeitspunkt der Gate-Spannung des Transistors V wird über den Spannungsteiler aus R2 und R3 eingestellt, bei dem der Widerstand $R_2$ Gate mit dem Versorgungspotential $V_{DD}$ verbindet, der Widerstand $R_3$ Gate mit einem weiteren Versorgungspotential, hier mit dem Massepotential.

Die Niederfrequenz-Kleinsignalverstärkung des Transistors V ergibt sich näherungsweise aus dem Produkt der Steilheit des Transistors $g_m$ und dem Widerstand $R_1$. Der vom Transistor V verstärkte Spannungsanteil ist das Produkt der Spannung der Signalquelle $V_{Analyt}$ mit dem Quotienten aus der Gate-Kapazität $C_G$ und der komplexen Summe der Kapazität der Elektrode $C_E$, der Gate-Kapazität $C_G$ und den Widerständen $R_2$ und $R_3$. Für nicht allzu niedrige Frequenzen nähert sich dieser Wert dem Quotienten aus $C_E$ und der Summe aus Gate-Kapazität $C_G$ und $C_E$ an. Die Gesamt-Niederfrequenz-Übertragungsfunktion $H_0$ ergibt sich dann zu:

$$H_0 = \left(\frac{V_{Out}}{V_{Analyt}}\right) \cong \left(\frac{C_G}{(C_E + C_G)}\right) \cdot g_m \cdot R_1. \tag{1}$$

Figur 2 zeigt ein zweites elektrisches Schaltbild einer erfindungsgemäßen Messzelle. Im Gegensatz zum Schaltbild nach Figur 1 verbindet das als Widerstand ausgebildete Lastelement $R_2$ Gate und Drain des Verstärkers V. Es dient als Rückkopplungswiderstand. Das Lastelement $R_3$ aus dem Schaltbild nach Figur 1 ist entbehrlich.

Bei dem Ausführungsbeispiel gemäß Figur 2 werden Betriebsparameterschwankungen - insbesondere prozessbedingte und lokale stochastische Betriebsparameterschwankungen - von Transistor V und Lastwiderstand $R_1$ bei der Arbeitspunktfestlegung berücksichtigt und automatisch kompensiert. Ferner ist der Platzbedarf auf dem Substrat durch eine geringere Anzahl von Widerständen gegenüber der Verstärkerschaltung B nach Figur 1 verringert.

Zur Betrachtung des sich einstellenden Arbeitspunktes werden Gleichgewichtsbedingungen betrachtet, d. h. zeitabhängige Signale vernachlässigt. Damit entspricht die Gate-Spannung der Drain-Spannung. Unter Vernachlässigung des Ausgangsleitwertes gilt für den Transistor V die konstituierende Gleichung:

$$I_{D0} = k \cdot (V_G - V_{Th})^2. \tag{2}$$

Aus der Maschenregel folgt für die Gate-Spannung:

$$V_G = V_{DD} - I_{D0} \cdot R_1. \tag{3}$$

Somit lässt sich die Gate-Spannung aus der konstituierenden Gleichung des Transistors eliminieren und der Drain-RuheStrom des Transistors V folgendermaßen angeben:

$$I_{D0} = \frac{1}{R_1} \cdot \left(V_{DD} - V_{Th} - \frac{1}{R_1 \cdot k} \cdot \left(\sqrt{1 + 2 \cdot R_1 \cdot k \cdot (V_{DD} - V_{Th})} - 1\right)\right). \tag{4}$$

Wie aus Gleichung (4) hervorgeht, nimmt der Ruhestrom des Transistors in etwa hyperbolisch mit zunehmendem Widerstand $R_1$ ab. Diese etwa hyperbolische Abnahme des Stromes, der über den Quotienten aus Strom und Spannung am Widerstand R1 gegeben ist, basiert in erster Linie auf der Zunahme des Teilers des Quotienten bei zunehmendem Widerstand.

Allerdings nimmt mit zunehmendem Widerstand auch der Spannungsabfall am Widerstand und somit der Zähler des Quotienten geringfügig zu, daher die Abweichung vom hyperbolischen Verlauf. Damit lässt sich über R1 der Ruhestrom, der die Steilheit $g_m$ bestimmt, einstellen.

Konkret ergibt sich mit den konstituierenden Gleichungen des Transistors eine schwächere als lineare Abnahme der die Verstärkung beeinflussenden Steilheit $g_m$. Dies ist wiederum auf die Abnahme der Gate- beziehungsweise Drain-Spannung und der daraus resultierenden Abnahme der Steilheit des Transistors mit zunehmendem Widerstand $R_1$ zurückzuführen (vgl. Gleichung (5)):

$$g_m = k \cdot \left( \frac{V_{DD} - V_{Th}}{\sqrt{1 + 2 \cdot R_1 \cdot k \cdot (V_{DD} - V_{Th})} - 1} - \frac{1}{R_1 \cdot k} \right). \qquad (5)$$

Der Effekt, auf dem die Kleinsignalverstärkung beruht, lässt sich relativ einfach erklären, wenn die Rückkopplung über den Widerstand $R_2$ zunächst vernachlässigt wird, die Kapazität der Elektrode im Grenzübergang als beliebig gut leitend und der Drain-Strom des Transistors in Figur 2 als allein von der Gate-Spannung abhängig betrachtet wird, also der Ausgangsleitwert vernachlässigt wird. Die Kleinsignalverstärkung ist der Quotient der Änderung der Ausgangsspannung zur Änderung der Eingangsspannung, also der Quotient der Änderung der Drain-Spannung zur Änderung der Gate-Spannung.

Eine geringe Änderung der Gate-Spannung $\Delta V_G$ hat eine Änderung des Drain-Stromes

$$\Delta I_D = -g_m \cdot \Delta V_G \qquad (6)$$

zur Folge.

Diese Stromänderung bewirkt eine Änderung des Spannungsabfalls am Widerstand bzw. Drain- Anschluss von $R_1 \cdot \Delta I_D$.

Damit ergibt sich in erster Näherung die Niederfrequenz-Kleinsignalverstärkung $h_0$ zu:

$$h_0 = \frac{\Delta V_D}{\Delta V_G} = g_m \cdot R_1. \qquad (7)$$

Wird der Ausgangsleitwert des Transistors berücksichtigt so sinkt die Verstärkung des Transistors nur unwesentlich ab, da der Ausgangsleitwert erheblich kleiner ist als der Reziprokwert des Widerstands $R_1$

Des weiteren interessiert auch der Frequenzgang der Schaltung. Minimalanforderung an den Frequenzgang ist die Übertragung der Frequenzanteile, welche in der zu betrachtenden Bandweite liegen. Wünschenswert ist gegebenenfalls die Dämpfung der Anteile in jenen Frequenzbereichen, die kein Nutzsignal enthalten, also ausschließlich das Signal-zu-Rausch-Verhältnis verschlechtern. Bei Betrachtung der Schaltung wird klar, dass der Frequenzgang der Schaltung von mehreren Faktoren bestimmt wird. Zum einen bildet die Kapazität der Elektrode mit den anderen Kapazitäten am Gate-Knoten einen kapazitiven Spannungsteiler.

Aufgrund der sehr kleinen Signale muss dieser Spannungsteiler geeignet dimensioniert sein, um eine weitere Abschwächung der Signale zu vermeiden. Dazu sollte die Kapazität $C_E$, mittels derer Sensor A und Gate verbunden werden, mindestens ähnlich groß sein wie die Gate-Kapazität. Die Gate-SpannungsÄnderungen werden am Drain-Knoten verstärkt und über das RC-Glied aus R2 und den Kapazitäten am Gate-Knoten wieder auf den Gate-Knoten rückgekoppelt. Da die Schaltung einen invertierenden Verstärker darstellt, ist diese Rückkopplung eine Gegenkopplung. Mit zunehmender Gegenkopplung sinkt die Verstärkung. Um dies im Durchlass-Frequenzbereich zu vermeiden, muss die Grenzfrequenz dieser Rückkopplung deutlich kleiner sein als die untere Soll-Grenzfrequenz des Verstärkers. Des weiteren wird der Frequenzgang auch noch von dem Ausgangswiderstand und der zu treibenden, parasitären Leitungskapazität beeinflusst, die der Verstärker treiben können muss.

Ein wesentlicher Effekt der Rückkopplung ist, dass der Arbeitspunkt der Schaltung gegenüber prozessbedingten Schwankungen, zum Beispiel der den Arbeitspunkt festlegenden Widerstände, im Vergleich zur Messzelle nach Figur 1 unempfindlicher wird, da der Gleichanteil der Gate-Spannung des Transistors über den Gleichanteil der Drain-Spannung festgelegt wird, und der Transistor somit immer einen Arbeitspunkt im Sättigungsbereich hat.

Bei der Messzelle nach Figur 3 ist gegenüber der Messzelle nach Figur 2 das weitere Lastelement $R_2$ durch einen steuerbaren Schalter $P_A$ ersetzt. Wird der Schalter $P_a$ geschlossen, so werden die am Gate befindlichen Kapazitäten auf die Spannung am Drain-Knoten aufgeladen und somit stellt sich ein Arbeitspunkt für die Drain-Spannung ein. Dieser Vorgang erfolgt in sich wiederholenden Einstellphasen, die zwischen Messbetriebsphasen liegen.

In den Messbetriebsphasen ist der steuerbare Schalter $P_a$ geöffnet und entspricht damit einem extrem hohen Rückkoppelwiderstand. Der Schalter $P_a$ lässt sich gut in Form eines Transistors realisieren. Im Betrieb wechseln sich Messbetriebsphasen und Einstellphasen ab, d.h. das Gesamtsystem arbeitet nicht vollkommen zeitkontinuierlich.

Wie bereits erwähnt ist für bestimmte Anwendungen die Anordnung der Messzellen in einem Feld sinnvoll. Das Feld besteht aus einzelnen Messzellen, auch Pixeln genant, die den Sensor, Verstärker, Verbindungselemente und gegebenenfalls Adressierungselemente enthalten. Soll eine Vielzahl von Sensoren aufgrund bereits erwähnter Notwendigkeiten oder aufgrund Wirtschaftlichkeitsgründen auf einer möglichst kleinen Fläche realisiert werden, so kann weder in jeder Messzelle ein vollständiger mehrstufiger Verstärker realisiert werden, noch kann jeder in einer Messzelle befindliche Vorverstärker fest mit einem außerhalb des Feldes befindlichen Verstärker verbunden sein, ohne den Flächenbedarf stark ansteigen zu lassen und dabei an Ortsauflösung zu verlieren.

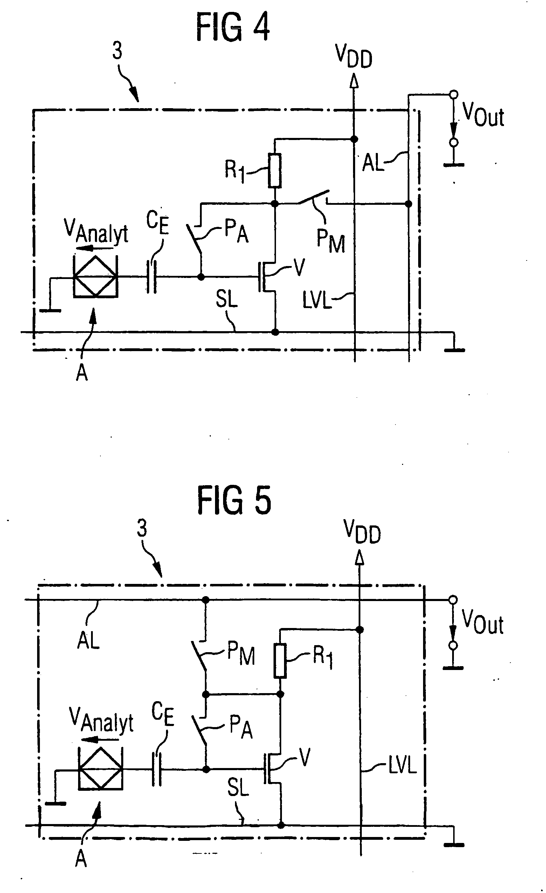

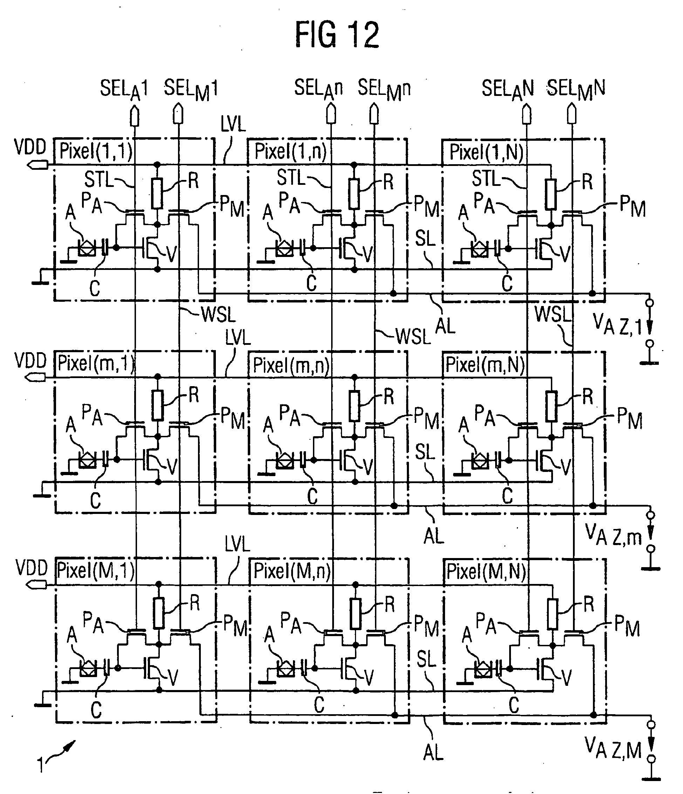

Anhand der Figuren 4 bis 9 werden unterschiedliche Messzellen zum Einsatz in einem Messfeld vorgeschlagen. Durch die Anordnung gleicher Messzellen neben- und untereinander entstehen in den Figuren 12 bis 17 dargestellte Messfelder.

Die Messzellen in den Figuren 4 bis 9 sind gepunktet umrissen; ebenso gepunktet umrissen sind die einzelnen Messzellen innerhalb der Messfelder. Dabei sind die Messzellen eines Messfeldes jedoch auf einem gemeinsamen Substrat aufgebracht. Der Umriß einer Messzelle nach einer der Figuren 4 bis 9 kann dabei auch die Substratgrenzen kennzeichnen. Bei Einsatz von Messzellen nach den Figuren 4 bis 9 in einem Messfeld kennzeichnet die gepunktete Linie aber gewöhnlich lediglich die Messzelle, nicht aber die Substratgrenze.

Die in Figur 4 dargestellte Messzelle ist ausgerichtet für ein Messfeld mit Messzellenreihen und Messzellenspalten, bei dem die Source-Anschlüsse zeilenweise mit dem Massepotential verbunden sind. Dazu dient eine sourceseitige Verbindungsleitung SL. Die Widerstände R1 werden spaltenweise fest mit dem Versorgungspotential $V_{DD}$ über eine lastseitige Versorgungsleitung LVL verbunden. Für diese mit Masse und dem Versorgungspotential $V_{DD}$ verbundenen Knoten ist im allgemeinen die Orientierung der Verbindungsleitungen beliebig. Das heißt, die sourceseitige Verbindungsleitung SL kann auch alle Zellen einer Spalte verbinden, die lastseitige Versorgungsleitung auch alle Zellen einer Reihe.

Der Transistor V arbeitet bei geöffnetem Schalter $P_A$ in Source-Schaltung, bewirkt also eine Verstärkung des Wechselanteils der Ersatzquelle $V_{Analyt}$.

Ferner ist ein weiterer steuerbarer Schalter $P_M$ vorgesehen. Wird der weitere Schalter $P_M$ zeilenweise ansteuerbar gewählt, so können die Ausgangssignale $V_{out}$ einzelner Messzellen zeilenweise ausgelesen werden. Dazu wird der weitere Schalter $P_M$ in den betreffenden Messzellen geschlossen, der Drain-Anschluss des Transistors V wird mit einer die Messzellen spaltenweise verbindenden Ausgangsleitung AL verbunden und das Ausgangssignal $V_{Out}$ kann dann am Rand des Messfeldes weiter verarbeitet werden.

Bei der Messzelle nach Figur 5 ist wiederum eine gemeinsame sourceseitige Verbindungsleitung SL zum Verbinden der Source-Anschlüsse einer Pixelzeile mit Masse vorgesehen. Auch die lastseitige Versorgungsleitung LVL entspricht derjenigen aus Figur 4. Dagegen ist nun eine Ausgangsleitung AL vorgesehen, die die weiteren Schalter $P_M$ aller Zellenreihen miteinander verbindet. Die Messzellen 3 können damit spaltenweise ausgelesen werden durch Ansteuern der weiteren steuerbaren Schalter $P_M$ einer Spalte.

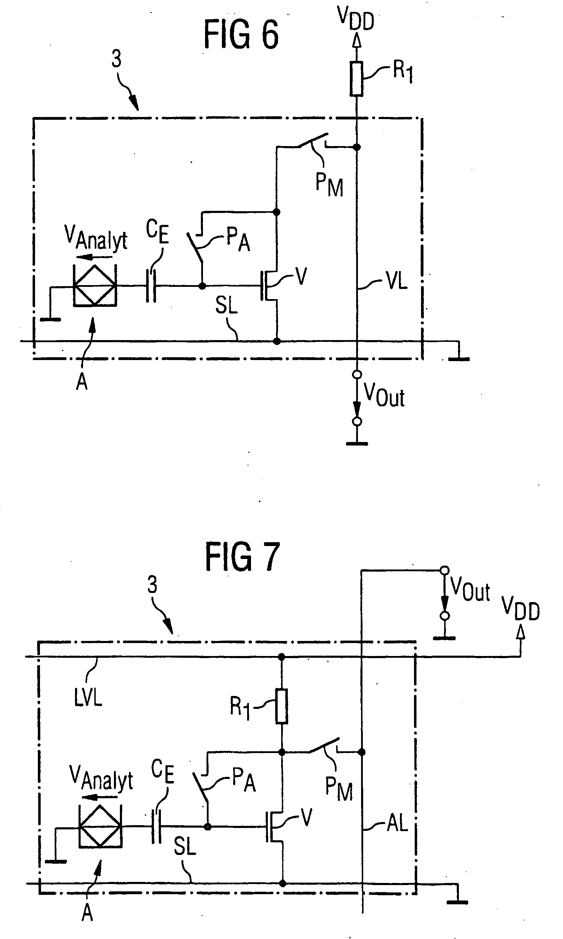

Gemäß Figur 6 kann das Lastelement $R_1$ auch außerhalb der Messzelle 3 angeordnet werden, d.h. hier auch außerhalb der Substrats, wenn der zeilenweise ansteuerbare weitere Schalter $P_M$ zwischen Lastelement $R_1$ und Transistor V angeordnet wird. Die Flächenausdehnung der Messzelle 3 ist reduziert, da der Flächenbedarf für einen Widerstand je Messzelle entfallen ist. Damit wird mit einem Messfeld mit Messzellen nach Figur 6 eine bessere Ortsauflösung erreicht. Des weiteren ist dann auch eine Regelung des Widerstands bei Einsatz eines veränderbaren Widerstandes und damit eine für jede Messzelle individuell einstellbare Verstärkung möglich.

Dabei ist wiederum eine gemeinsame sourceseitige Verbindungsleitung SL zum Verbinden der Source-Anschlüsse einer Pixelzeile mit Masse vorgesehen. Ferner ist eine Verbindungsleitung VL zwischen dem Lastelement $R_1$ und dem Ausgangssignalabgriff $V_{Out}$ vorgesehen. An diese Verbindungsleitung VL ist der weitere steuerbare Schalter $P_M$ angeschlossen. Bei der Anordnung weiterer derartiger Messzellen nach Figur 6 zu einem Messfeld werden alle weiteren steuerbaren Schalter $P_M$ einer Spalte an die Verbindungsleitung angeschlossen. Durch Ansteuern der weiteren steuerbaren Schalter $P_M$ einer Zeile sind damit die Messzellen zeilenweise auslesbar.

Bei der Messzelle nach Figur 7 entspricht im wesentlichen der Messzelle nach Figur 4. Es ist wiederum eine gemeinsame sourceseitige Verbindungsleitung SL zum Verbinden der Source-Anschlüsse einer Pixelzeile mit Masse vorgesehen. Ferner ist wiederum eine Ausgangsleitung vorgesehen, die die weiteren steuerbaren Schalter $P_M$ aller Zellenreihen miteinander verbindet. Die Messzellen können damit zeilenweise ausgelesen werden durch Ansteuern der weiteren steuerbaren Schalter $P_M$ einer Zeile. Die lastseitige Versorgungsleitung LVL ist nun aber nicht zum Verbinden der Lastelemente $R_1$ einer Zellenspalte sondern zum Verbinden der Lastelemente $R_1$ einer Zellenreihe vorgesehen.

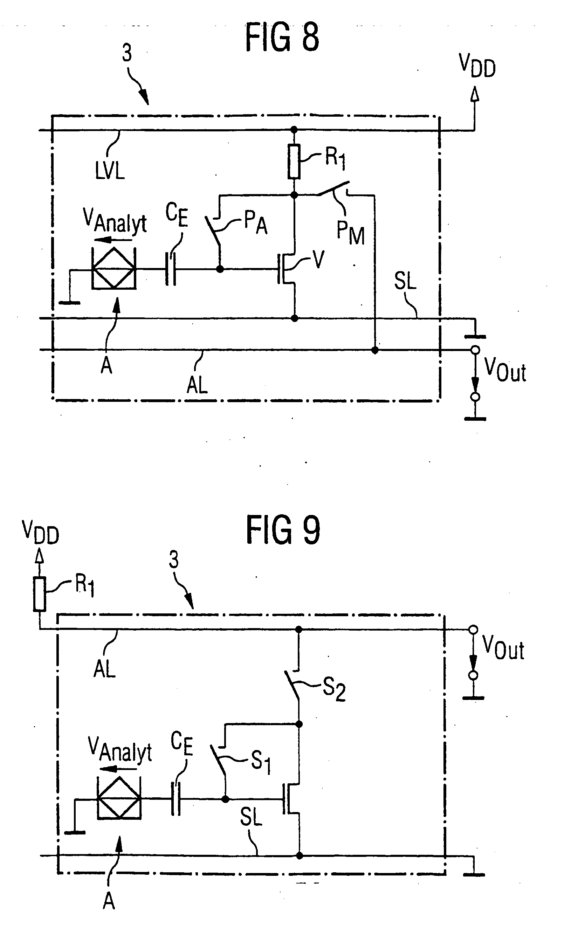

Gegenüber der Messzelle nach Figur 7 ist bei der Messzelle nach Figur 8 nun die Ausgangsleitung AL zum Verbinden von weiteren steuerbaren Schaltern $P_M$ einer Zellenreihe vorgesehen, gegenüber dem Verbinden einer Zellenspalte nach Figur 7. Die Messzellen können damit spaltenweise ausgelesen werden durch Ansteuern der weiteren steuerbaren Schalter $P_M$ einer Spalte.

Figur 9 zeigt im Grundsatz eine Messzelle nach Figur 6 mit einem externen Lastelement $R_1$. Dabei ist die

Verbindungsleitung aber vorgesehen zum Anschließen aller weiteren steuerbaren Schalter $P_M$ einer Zeile, nicht einer Spalte gemäß Figur 6. Durch Ansteuern der weiteren steuerbaren Schalter $P_M$ einer Spalte sind damit die Messzellen zeilenweise auslesbar. Zu den Vorteilen einer solchen Anordnung wird auf die Ausführungen zu Figur 6 verwiesen.

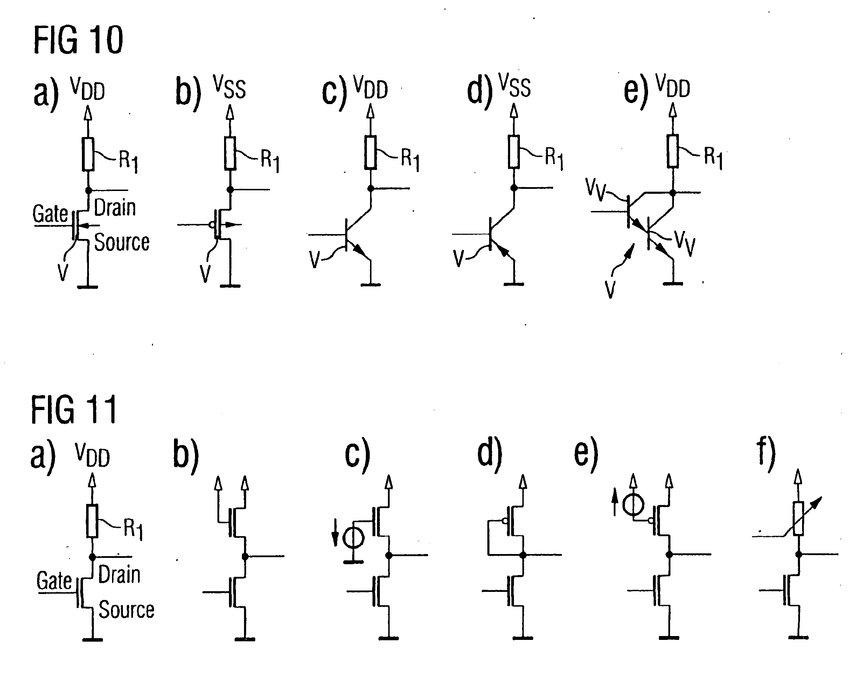

Figur 10 a) bis 10 e) zeigt einen Ausschnitt der erfindungsgemäßen Verstärkerschaltung mit je einem Lastelement $R_1$ und einem Transistorelement V zwischen dem Versorgungspotential $V_{DD}$ und dem Massepotential. Die Darstellungen dienen dazu, die alternative Verwendung unterschiedlicher Transistorelemente V als ein wesentliches Element der erfindungsgemäßen Verstärkerschaltung aufzuzeigen.

Dabei zeigt Figur 10 a) die Verwendung eines n-Typ-Feldeffekttransistors, Figur 10 b) die Verwendung eines p-Typ-Feldeffekttransistors, beide in Source-Schaltung, Figur 10 c) die Verwendung eines npn-Typ-Bipolartransistors, Figur 10 d) die Verwendung pnp-Typ-Bipolartransistors, beide in Emitter-Schaltung, und Figur 10 e) die Verwendung einer Darlingtonschaltung enthaltend zwei Subtransistoren VV.

Bei allen in den übrigen Figuren vorgestellten Schaltungsvarianten kommen n-Typ-MOSFETs, wie in Abb. 10 a) skizziert, zum Einsatz.

Bei allen Schaltungsvarianten können sowohl in Bezug auf verstärkende wie auch in Bezug auf schaltende Transistoren aber auch p-Typ-MOSFETs nach Figur 10 b), npn-Typ-Bipolartransistoren nach Figur 10 c), pnp-Typ-Bipolartransistoren nach Figur 10 d) oder ähnliche Verstärker-Bauelemente verwendet werden.

Grundsätzlich ist auch eine Kaskadierung gemäß der in Figur 10 e) dargestellten Darlingtonschaltung denkbar. Bei allen bipolaren Varianten ist eine zeitdiskrete Gegenkopplung nicht möglich. Eine zeitkontinuierliche Gegenkopplung mit einem Widerstand zwischen Kollektor- und Basisknoten hingegen ist möglich, da der für den Betrieb der Schaltung notwendige Basisstrom über einen solchen Widerstand geliefert wird.

Figur 11 a) bis 11 e) zeigt einen Ausschnitt der erfindungsgemäßen Verstärkerschaltung mit je einem Lastelement $R_1$ und einem n-Typ-Feldeffekttransistor V in Source-Schaltung zwischen dem Versorgungspotential $V_{DD}$ und dem Massepotential.

Die Darstellungen dienen dazu, die alternative Verwendung unterschiedlicher Lastelemente $R_1$ aufzuzeigen. Dabei zeigt Figur 10 a) die Verwendung eines ohmschen Widerstandes als Lastelement $R_1$, Figur 11 b) die Lastelements $R_1$ in Form eines Transistors vom gleichen Leitfähigkeitstyp wie der Verstärkertransistor V, dessen Gate-Spannung identisch mit der Drain-Spannung ist, Figur 11 c) die Verwendung eines Transistors nach Figur 11 b), dessen Gate-Spannung durch eine Spannungsquelle festgelegt wird, Figur 11 d) die Verwendung eines Transistor vom komplementären Leitfähigkeitstyp des Verstärkertransistors V, dessen Gate mit dem Drain verbunden ist, nach Figur 11 e) die Verwendung eines Transistors nach Figur 11 d), dessen Gate-Spannung durch eine Spannungsquelle festgelegt wird, und nach Figur 11 f) die Verwendung einer anderen Realisierung eines regelbaren oder nicht regelbaren Lastelements.

Bei allen in den übrigen Figuren vorgestellten Schaltungsvarianten kommen ohmsche Widerstände als Lastelemente, wie in Abb. 10 a) skizziert, zum Einsatz. Wie bereits diskutiert ergibt sich die Niederfrequenz-Kleinsignalverstärkung in diesem Fall aus dem Produkt von Steilheit des verstärkenden Transistors und dem Betrag des Widerstandes.

Bei allen Schaltungsvarianten kann aber auch ein Lastelement in Form eines Transistors vom gleichen Leitfähigkeitstyp gemäß Figur 11 b) verwendet werden. Da Drain-Spannung und Gate-Spannung identisch sind, ergibt sich ein Arbeitspunkt im Sättigungsbereich bzw. ein etwa quadratischer Zusammenhang zwischen Strom und Spannung, der jedoch im Arbeitsbereich in erster Näherung der Charakteristik eines Widerstandes entspricht.

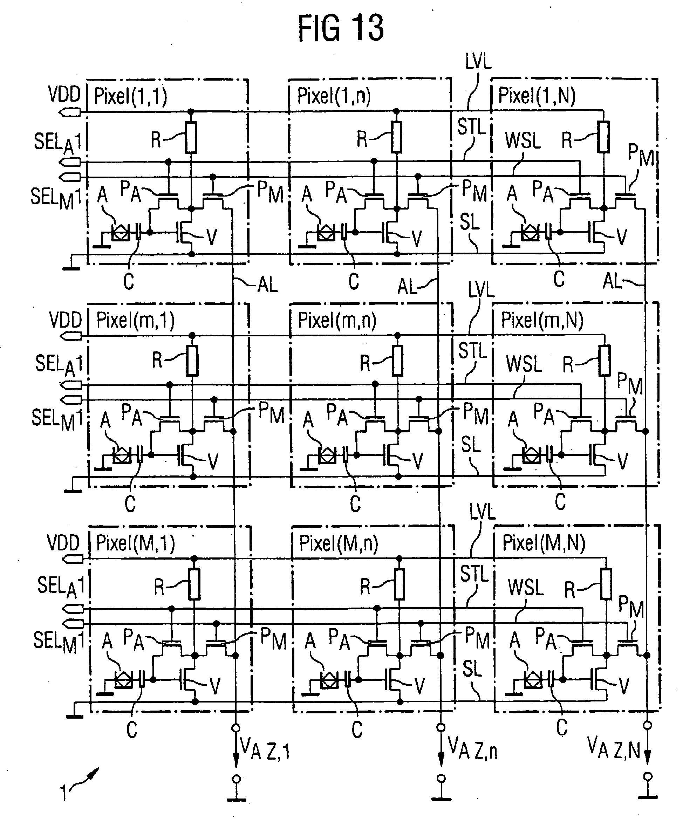

Letztendlich ergibt sich die Niederfrequenz-Kleinsignalverstärkung in etwa aus dem Quotienten der Steilheit des unteren und des oberen Transistors. Alternativ dazu kann ein Transistor des komplementären Leitfähigkeitstyps wie gemäß Figur 11 d) verwendet werden oder die Gate-Spannung auch über eine Spannungsquelle festgelegt werden wie in den Figuren 11 c) oder 11 e) dargestellt. Prinzipiell eignet sich jede andere Realisierungsvariante eines nicht regelbaren oder regelbaren Lastelements gemäß Figur 13 f).

Als Beispiel eines regelbaren Widerstandes sei hier auf die Veröffentlichung [9] verwiesen.

Dann wird auch die Verstärkung der Verstärker in den einzelnen Pixeln regelbar.

Es sei an dieser Stelle noch einmal ausdrücklich erwähnt, dass alle beschrieben Widerstandselemente, zum Beispiel auch der Spannungsteiler $R_2/R_3$ aus Figur 1 mit anderen Lastelementen als ohmschen Widerständen realisiert werden können.

Figur 12 zeigt ein Messfeld mit 3x3 Messzellen, in der Figur auch mit dem Begriff "Pixel" beschriftet. Die verwendeten Messzellen 3 entsprechen dem Messzellenaufbau nach Figur 8.

Die Rückstell- bzw. Einstellfunktion des Schalters $P_A$ wird mittels des Schalt- bzw. Pass- Transistors realisiert und der weitere Schalter $P_M$ zur Messung mittels eines Schalttransistors. Prinzipiell können diese Transistoren auch vom komplementären Leitfähigkeitstyp sein, jedoch wird dann die Fläche aufgrund der erforderlichen Wannen deutlich größer.

Über die horizontalen und damit Pixelreihen verbindenden Source-Verbindungsleitungen SL und lastseitigen Verbindungsleitungen LVL werden Massepotential beziehungsweise erstes Versorgungspotential $V_{DD}$ an die Lastelemente R und die Source-Anschlüsse angelegt. Damit fällt die Potentialdifferenz dieser Potentiale, die sich auch von der Betriebspannung des Chips unterscheiden kann an der Serienschaltung aus dem Widerstand R und dem Transistor V ab.

Während der Einstellphase, die einer Messphase vorausgeht, wird der Arbeitspunkt der Verstärker V in den einzelnen Pixeln eingestellt. Dazu werden alle Schalter $P_A$ durchgeschaltet, indem an die Anschluss $SEL_A$ der Steuerleitungen STL für die Schalter $P_A$ die Betriebspannung angelegt wird. Je eine Steuerleitung S1 verbindet alle Steueranschlüsse der Schalter $P_A$ einer Pixelspalte. Über den Schalter $P_A$ sind somit Gate und Drain des Transistors V kurzgeschlossen und es stellt sich ein stabiler Arbeitspunkt in allen Pixeln ein. Dieser liegt im Sättigungsbereich, da die Spannung an der Gate-Source-Kapazität gleich der Drain-Source-Spannung ist. Am Ende der Einstellphase werden die Transistoren $P_A$ wieder hochohmig geschaltet. Der Transistor V verstärkt nun.

Eine Veränderung der untersuchten Eigenschaft der Probe führt zu einer Spannungsänderung am Sensor und damit an der Gate-Kapazität des Transistors V beziehungsweise einer Änderung der Gate-Spannung, welche eine ungefähr um den Faktor

$$- R \cdot g_m \qquad\qquad\qquad\qquad\qquad (8)$$

verstärkte Änderung der Drain-Spannung zur Folge hat.

Diese Drain-Spannung kann in der in Figur 12 dargestellten Konfiguration gleichzeitig bzw. parallel für eine Pixelspalte n ausgelesen werden. Dazu werden alle Schalter $P_A$, welche sich nicht in der n-ten Spalte befinden, gesperrt. Die entsprechenden n- Typ Transistoren werden dazu mit einem Low-Pegel an den Anschlüssen $SEL_M$ der die weiteren Schalter $P_M$ einer Pixelspalte verbindenden weiteren Steuerleitungen WSL gesteuert. Die weiteren Schalter $P_M$ der n-ten Spalte werden durchgeschaltet. Damit liegen die Drain-Spannungen $V_A$ der Transistoren V in der n-ten Spalte an horizontal verlaufenden Ausgangsleitungen AL an und können ausgelesen werden.

Eine bidirektionale Signalübertragung eines Impulses auf eine Probe ist insofern möglich, als nicht nur die Pixel ausgelesen werden können sondern auch eine gezielte Übermittlung von Signalen an einzelne Sensoren möglich wird. Ein solcher Impuls kann beispielsweise über die m-te Ausgangsleitung AL bei durchgeschalteten Schaltern $P_A$ und $P_M$ an den als Sensor dienenden Feldeffekttransistor der Messzelle geleitet werden.

Figur 13 zeigt ein Messfeld mit 3x3 Messzellen, in der Figur auch mit dem Begriff "Pixel" beschriftet. Die verwendeten Messzellen 3 entsprechen dem Messzellenaufbau nach Figur 7.

Über die horizontalen und damit Pixelreihen verbindenden Source-Verbindungsleitungen SL und lastseitigen Verbindungsleitungen LVL werden Massepotential beziehungsweise erstes Versorgungspotential $V_{DD}$ an die Lastelemente R und die Source-Anschlüsse angelegt. Damit fällt die Potentialdifferenz dieser Potentiale, die sich auch von der Betriebspannung des Chips unterscheiden kann an der Serienschaltung aus dem Widerstand R und dem Transistor V ab.

Die Schalter $P_A$ einer Pixelreihe werden über gemeinsame Steuerleitungen STL je Pixelreihe miteinander verbunden. Die weiteren Schalter $P_M$ einer Pixelreihe werden über gemeinsame weitere Steuerleitungen WSL je Pixelreihe miteinander verbunden.

Die weiteren Schalter $P_M$ einer jeden Spalte sind über vertikal verlaufende Ausgangsleitungen AL miteinander verbunden.

Zur Betriebsweise wird auf die Ausführungen bezüglich des Messfeldes nach Figur 12 verwiesen. Im Unterschied hierzu können jedoch bei dem Messfeld nach Figur 13 die Pixel zeilenweise ausgelesen werden.

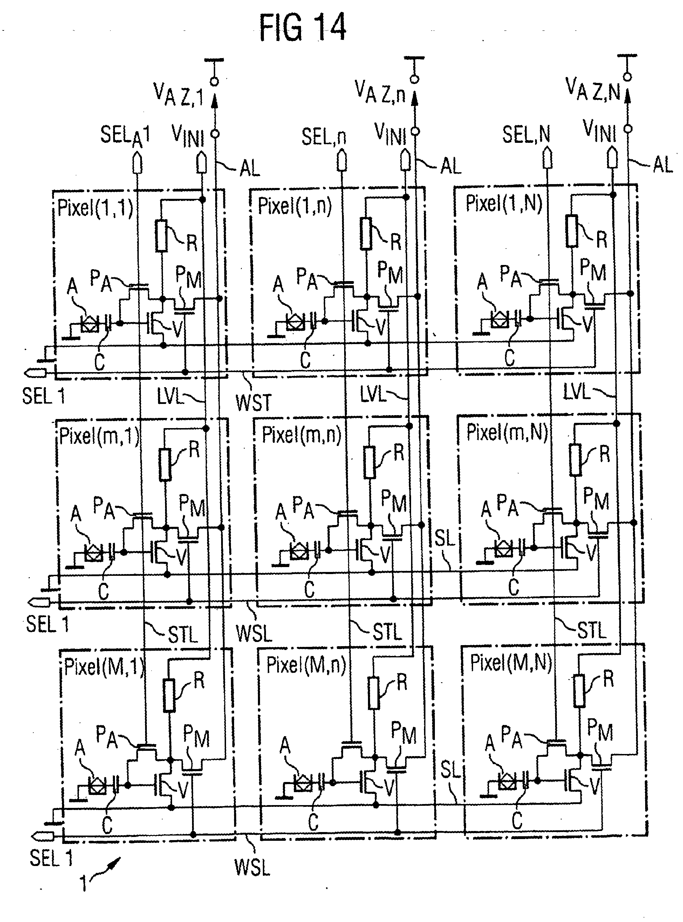

Figur 14 zeigt ein Messfeld mit 3x3 Messzellen, in der Figur auch mit dem Begriff "Pixel" beschriftet. Die verwendeten Messzellen 3 entsprechen dem Messzellenaufbau nach Figur 4.

Über die horizontalen und damit Pixelreihen verbindenden Source-Verbindungsleitungen SL und die vertikalen und damit Pixelspalten verbindenden lastseitigen Verbindungsleitungen LVL werden Massepotential beziehungsweise erstes Versorgungspotential $V_{DD}$ an die Lastelemente R und die Source-Anschlüsse angelegt. Damit fällt die Potentialdifferenz dieser Potentiale, die sich auch von der Betriebspannung des Chips unterscheiden kann an der Serienschaltung aus dem Widerstand R und dem Transistor V ab.

Die Schalter $P_A$ einer Pixelspalte werden über gemeinsame Steuerleitungen STL je Pixelspalte miteinander verbunden. Die weiteren Schalter $P_M$ einer Pixelreihe werden über gemeinsame weitere Steuerleitungen WSL je Pixelreihe miteinander verbunden.

Die weiteren Schalter $P_M$ einer jeden Spalte sind über vertikal verlaufende Ausgangsleitungen AL miteinander verbunden.

Zur Betriebsweise wird auf die Ausführungen bezüglich des Messfeldes nach Figur 12 verwiesen. Bei dem Messfeld nach Figur 14 werden die Pixel zeilenweise ausgelesen werden.

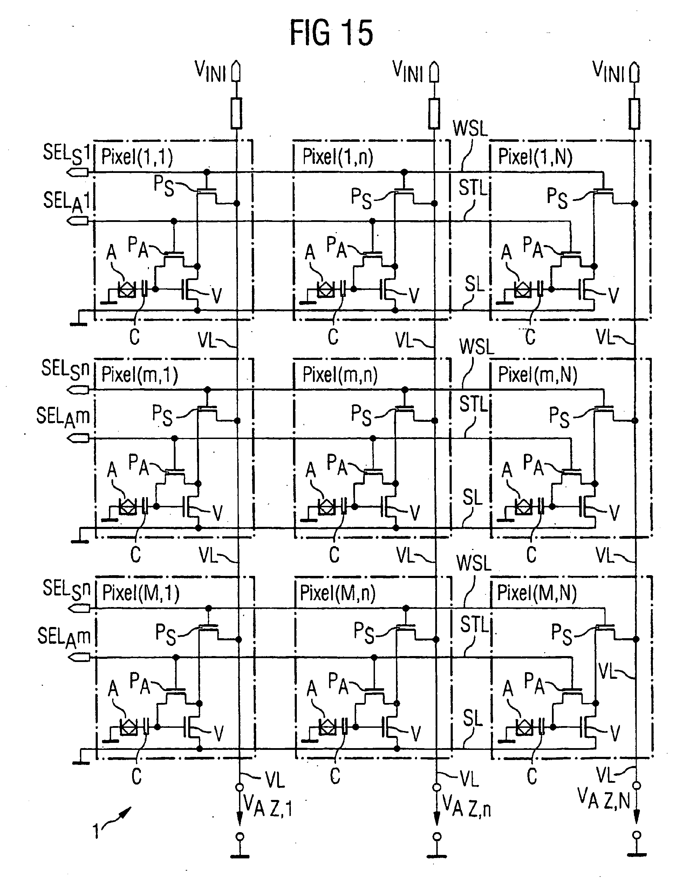

Figur 15 zeigt ein Messfeld mit 3x3 Messzellen, in der Figur auch mit dem Begriff "Pixel" beschriftet. Die verwendeten Messzellen 3 entsprechen dem Messzellenaufbau nach Figur 6.

Über die horizontalen und damit Pixelreihen verbindenden Source-Verbindungsleitungen SL wird Massepotential an die Source-Anschlüsse angelegt. Je Pixelspalte ist nun nur ein, außerhalb des Substrats angeordneter Widerstand als Lastelement R vorgesehen.

Dieses Lastelement R ist mit dem ersten Versorgungspotential $V_{DD}$ verbunden. Die weiteren Schalter $P_S$ einer Pixelspalte sind über eine Verbindungsleitung VL mit dem dieser Spalte zugeordneten Lastelement R einerseits und andererseits mit einem Abgriff für das Ausgangssignal $V_A$ verbunden.

Die Schalter $P_A$ einer Pixelreihe werden über gemeinsame Steuerleitungen STL je Pixelreihe miteinander verbunden. Die weiteren Schalter $P_S$ einer Pixelreihe werden über gemeinsame weitere Steuerleitungen WSL je Pixelreihe miteinander verbunden.

Bis auf jene weiteren Schalter $P_S$ der auszulesenden Zeile werden alle weiteren Schalter $P_S$ mit einem Low-Pegel angesteuert und somit gesperrt. Die mit einem High-Pegel durchgeschalteten weiteren Schalter $P_S$ verhalten sich ohmsch oder arbeiten im Triodenbereich. Damit bilden die Transistoren V - mit der Steilheit $g_m$ und dem Ausgangsleitwert $g_d$ - mit den Widerständen R und den durchgesteuerten weiteren als Schalttransistoren ausgebildeten Schaltern $P_S$ - mit dem Ausgangsleitwert $g_{dps}$ - einen Verstärker mit der Verstärkung der Gate-Spannung $V_G$ am Ausgang $V_A$ um den Faktor $A_0$:

$$A_0 = \frac{\Delta V_A}{\Delta V_G} = R_1 \cdot g_m \cdot \frac{1}{1 + g_d \cdot \left(R_1 + \frac{1}{g_{dP_S}}\right)} \cdot \qquad (9)$$

Bei dem Messfeld nach Figur 15 werden die Pixel zeilenweise über den stromfreien Teil der Verbindungsleitung VL ausgelesen.

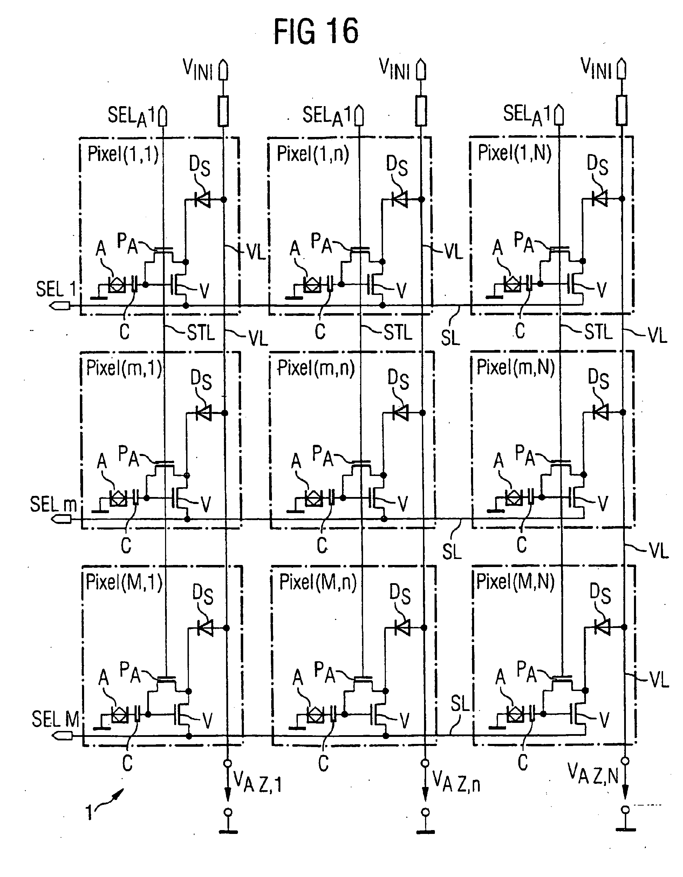

Figur 16 zeigt ein Messfeld mit 3x3 Messzellen, in der Figur auch mit dem Begriff "Pixel" beschriftet. Die verwendeten Messzellen 3 entsprechen im wesentlichen dem Messzellenaufbau nach Figur 6.

Über die horizontalen und damit Pixelreihen verbindenden Source-Verbindungsleitungen SL wird Massepotential an die Source-Anschlüsse angelegt. Je Pixelspalte ist nun nur ein, außerhalb des Substrats angeordneter Widerstand als Lastelement R vorgesehen. Dieses Lastelement R ist dem ersten Versorgungspotential $V_{DD}$ verbunden. Die weiteren Schalter $P_M$ aus Figur 6 sind durch eine Diode ersetzt. Die Dioden $D_S$ einer Pixelspalte sind über eine Verbindungsleitung VL mit dem dieser Spalte zugeordneten Lastelement R einerseits und andererseits mit einem Abgriff für das Ausgangssignal $V_A$ verbunden.

Die Schalter $P_A$ einer Pixelspalte werden über gemeinsame Steuerleitungen STL je Pixelreihe miteinander verbunden.

Eine Ansteuerung oder Auswahl von einer Zeile von Messzellen wird vorzugsweise ermöglicht, indem die Verbindungsleitung SL der angesteuerten Zeile auf Massepotential gelegt wird und alle anderen horizontalen Verbindungsleitungen SL auf das positive Versorgungspotential $V_{DD}$ gelegt werden. Die Dioden $D_S$ in den nicht angesteuerten Messzellen sperren dann, da die Dioden $D_S$ in diesen Zeilen in Sperrrichtung gepolt sind. Die in der angesteuerten Spalte befindlichen Dioden sind in Flussrichtung gepolt und stellen somit einen kleinen Widerstand dar, der im Vergleich zum Widerstand des Lastelements R vernachlässigt werden kann.

Bei dem Messfeld nach Figur 16 werden die Pixel somit zeilenweise ausgelesen.

In einer alternativen vorteilhaften Ausführungsvariante kann das Messfeld auch spaltenweise ausgelesen werden.

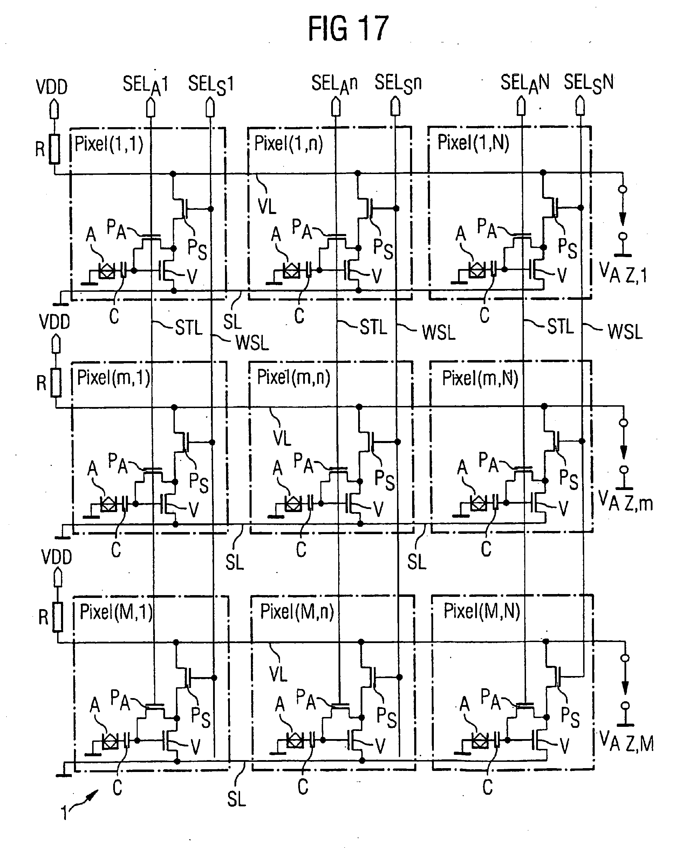

Figur 17 zeigt ein Messfeld mit 3x3 Messzellen, in der Figur auch mit dem Begriff "Pixel" beschriftet. Die verwendeten Messzellen 3 entsprechen dem Messzellenaufbau nach Figur 9.

Über die horizontalen und damit Pixelreihen verbindenden Source-Verbindungsleitungen SL wird Massepotential an die Source-Anschlüsse angelegt. Je Pixelreihe ist nun nur ein, außerhalb des Substrats angeordneter Widerstand als Lastelement R vorgesehen. Dieses Lastelement R ist dem ersten Versorgungspotential $V_{DD}$ verbunden. Die weiteren Schalter $P_M$ einer Pixelreihe sind über eine Verbindungsleitung VL mit dem dieser Spalte zugeordneten Lastelement R einerseits und andererseits mit einem Abgriff für das Ausgangssignal $V_A$ verbunden.

Die Schalter $P_A$ einer Pixelspalte werden über gemeinsame Steuerleitungen STL je Pixelspalte miteinander verbunden. Die weiteren Schalter $P_M$ einer Pixelspalte werden über gemeinsame weitere Steuerleitungen WSL je Pixelspalte miteinander verbunden.

Zur Betriebsweise wird auf die Ausführungen bezüglich des Messfeldes nach Figur 12 verwiesen. Bei dem Messfeld nach Figur 17 werden die Pixel spaltenweise ausgelesen.

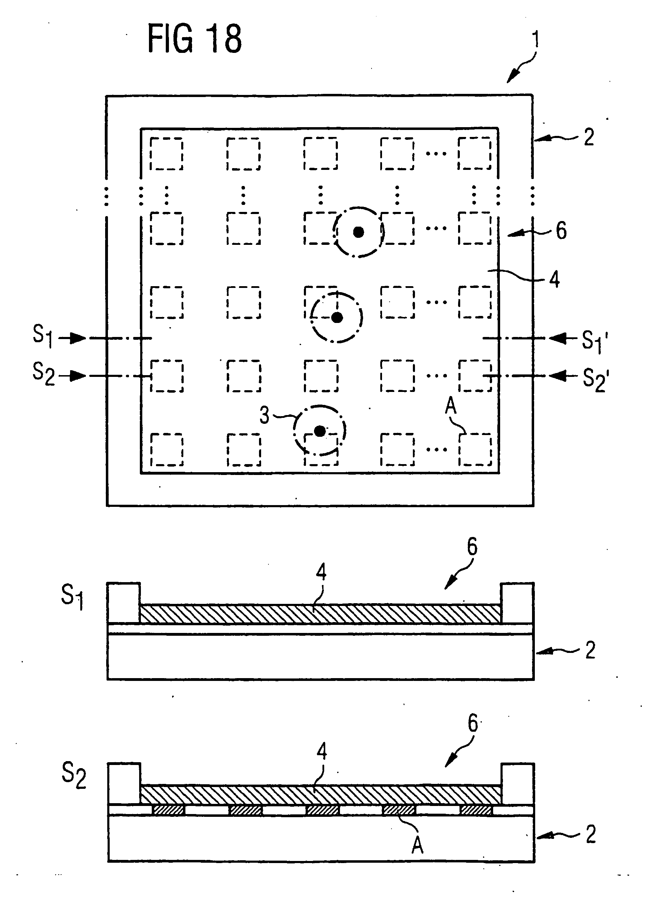

Figur 18 zeigt ein Messfeld 1 in Draufsicht. Die unter der Draufsicht gezeigten Ansichten sind Schnitte entlang der Schnittlinien S1 - S1' und S2 - S2' aus der Draufsicht.

Dabei bilden die Messzellen 3 den Boden einer Wanne 6. Die Wanne 6 nimmt im Messbetrieb die Probe 4 / den Analyten auf. Damit kann die Probe auf die Sensoren A der Messzellen 3 einwirken. Ferner ist das Substrat 2 ersichtlich, das die Messzellen 3 trägt und bei dem vorliegenden Ausführungsbeispiel ein Siliziumchip ist, der die in CMOS-Technik aufgebauten Sensoren und Verstärkerschaltungen trägt.

Die einzelnen Sensoren können dabei entweder gleich aufgebaut sein und elektrische Potentiale der Probe ortsaufgelöst messen. Die Sensoren können aber auch unterschiedlich aufgebaut sein zur Messung unterschiedlicher Parameter /Substanzen der Probe.

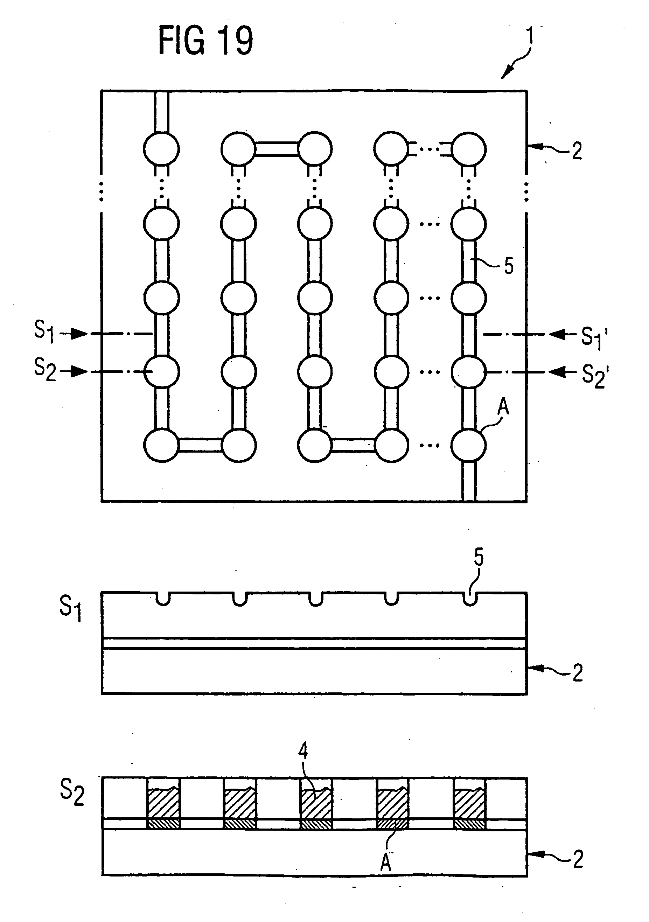

Figur 19 zeigt ein weiteres Messfeld in Draufsicht und darunter zwei Schnittbilder entlang der Schnittlinien S1 - S1' und S2 - S2' aus der Draufsicht. Dabei nimmt nicht eine Wanne die Probe auf sondern ein Kanal 5 führt die Probe im Rahmen einer Fluidik an den einzelnen Sensoren A der Messzellen 3 vorbei.

Auch hier können die einzelnen Sensoren entweder gleich aufgebaut sein und elektrische Potentiale der Probe ortsaufgelöst messen. Die Sensoren können aber auch unterschiedlich aufgebaut sein zur Messung unterschiedlicher Parameter / Substanzen der Probe.

In diesem Dokument ist folgende Veröffentlichung zitiert:

[1] K. Takahashi, S. Takeuchi, Bi-MOSFET Amplifier for Integration with Multimicroelectrode Array for Extracellular Neuronal Recording, IEICE TRANS. FUNDAMENTALS, Vol. E77-A, No. 2, 388 - 393, Feb. 1994

[2] P. Fromherz, Interfacing Neurons and Silicon by Electrical Induction, Ber. Bunsenges. Phys. Chem. 100, No. 7, 1093 - 1102, 1996

[3] A. Stett et al., Two-way silicon-neuron interface by electrical induction, Physical Review E, Volume 55, Number 2, 1779 - 1782, Feb. 1997

[4] S. Vassanelli, P. Fromherz, Transistor records of excitable neurons from rat brain, Appl. Phys. A66 459 - 463, 1998

[5] W.J. Parak et al., The field-effect-addressable potentiometric sensor/stimulator (FAPS) - a new concept for a surface potential sensor and stimulator with spatial resolution, Sensors and Actuators, B-58, 497 - 504, 1999

[6] T. C. W. Yeow et al., Design of a Single Cell of an ISFET Array Chip, Microelectronics: Technology today for the future, NA ( NA) 62 - 67, 1995

[7] T. C. W. Yeow et al., A very large integrated pH-ISFET sensor array chip compatible with standard CMOS process, Sensors an Actuators B 44, 434 - 440, 1997

[8] T. C. W. Yeow et al., Thick-Film Thermistor Array Using a Novel Threshold Conversion Concept, Sensors an Materials, Vol. 10, No. 2, 77 - 91, 1998

[9] K. Vavelidis, Y. Tsividis, IEEE International Symposium on Circuits and Systems, New York, 0-7803-1254-6/93, 1180 - 1183, 1993

[10] EP 0 942 259 A1

[11] US 5 309 085

[12] US 5 965 452

[13] O. Limann, Elektronik ohne Ballast: Einführung in die Schaltungstechnik der industriellen Elektronik, Franzis-Verlag, 7. Auflage, S. 35 - 38, 1987

[14] K. Tsukada, T. Maruizumi, H. Miyagi, ISFET Incorporated in a Differential Amplifier, Electronics and Communications in Japan, Part 2, Vol. 71, No. 3, S. 82 - 84, 1988

**Bezugszeichenliste**

S1-S1'     Schnittlinie
S2-S2'     Schnittlinie

| 1 | Meßfeld |
|---|---|
| S | Spalte |
| R | Reihe |
| SL | Source-Verbindungsleitung |
| STL | Steuerleitung |
| WSL | Weitere Steuerleitung |
| AL | Ausgangsleitung |
| LVL | Lastseitige Versorgungsleitung |
| VL | Verbindungsleitung |
| 2 | Substrat |
| 3 | Meßzelle |
| A | Sensor |
| B | Verstärkerschaltung |
| V | Transistor |
| VV | Subtransistor |
| $P_A$ | steuerbarer Schalter |
| $P_M$ $P_S$ | weiterer steuerbarer Schalter |
| R, $R_1$ | Lastelement |
| $R_2$ | Weiteres Lastelement |
| $R_3$ | Drittes Lastelement |
| $V_{DD}$ | Erstes Versorgungspotential |
| Massezeichen | Zweites Versorgungspotential |
| $V_{Analyt}$ | elektrisches Wandlersignal |
| $V_{Out}$ | Elektrisches Ausgangssignal |
| C, $C_E$ | Kapazität |
| 4 | Probe |
| 5 | Kanal |
| 6 | Wanne |

**Patentansprüche**

1. Messzelle (3) zum Aufnehmen eines elektrischen Potentials eines sich auf der Messzelle (3) befindenden Analyten,

• mit einem Wandlerelement (A), welches eingerichtet ist zum Umsetzen einer Messgröße in ein elektrisches Potential, wobei das Wandlerelement (A) eine Elektrode aufweist;
• mit einer Verstärkerschaltung (B), welche über eine Entkopplungskapazität ($C_E$) mit dem Wandlerelement (A) verbunden ist, wobei die Verstärkerschaltung (B) eine Eingangsstufe aufweist, wobei die Eingangsstufe der Verstärkerschaltung (B) einen Feldeffekttransistor (V) oder einen Bipolartransistor (V) enthält, und wobei die Elektrode zumindest mittelbar mit einem Steueranschluss des Feldeffekttransistors (V) bzw. des Bipolartransistors (V) der Eingängsstufe der Verstärkerschaltung (B) verbunden ist; und
• mit einer Arbeitspunkt-Einstelleinheit ($R_1$, $R_2$, $P_A$, $P_M$) zum Einstellen eines geeigneten Arbeitspunktes der Verstärkerschaltung (B), wobei die Arbeitspunkt-Einstelleinheit derart eingerichtet ist, dass die Einstellung eines geeigneten Arbeitspunktes der Verstärkerschaltung (B) erfolgt über die Einprägung einer geeigneten Spannung oder eines geeigneten Stromes an dem Steueranschluss des Feldeffekttransistors (V) bzw. des Bipolartransistors (V) der Eingangsstufe der Verstärkerschaltung (B), und wobei die Verstärkerschaltung (B) eine Rückkopplung aufweist, über welche die Einprägung der Spannung bzw. des Stromes an dem Steueranschluss des Feldeffekttransistors (V) bzw. des Bipolartransistors (V) erfolgt, derart, dass Parameterschwankungen der Verstärkerschaltung (B) automatisch kompensiert werden; und
• bei der die Elektrode und die Verstärkerschaltung (B) auf einem gemeinsamen Substrat (2) integriert angeordnet sind.

2. Messzelle (3) nach Anspruch 1,
bei der die Verstärkerschaltung (B) einstufig ausgestaltet ist.

3. Messzelle (3) nach Anspruch 1,
bei der die Verstärkerschaltung (B) mehrstufig ausgestaltet ist.

**4.** Messzelle (3).nach einem der Ansprüche 1 bis 3,
bei der die Rückkopplung eingerichtet ist als

• ein gesteuerter Schalter ($P_A$),
• ein Tiefpass oder
• eine Einheit zur externen Abspeicherung einer Betriebsspannung.

**5.** Messzelle (3) nach einem der Ansprüche 1 bis 4,

• bei der zum Einstellen des Arbeitspunktes des Feldeffekttransistors (V) bzw. des Bipolartransistors (V) zumindest ein Lastelement ($R_1$) vorgesehen ist,
• bei der Drain beziehungsweise der Kollektor des Transistors (V) über das Lastelement ($R_1$) mit einem ersten Versorgungspotential verbunden ist, und
• bei der der Steueranschluss des Feldeffekttransistors (V) bzw. des Bipolartransistors (V) über einen steuerbaren Schalter ($P_A$) mit Drain beziehungsweise dem Kollektor des Transistors (V) verbunden ist.

**6.** Messzelle (3) nach einem der Ansprüche 1 bis 5,
bei der der Transistor (V) als ein in Source-Schaltung betriebener Feldeffekttransistor ausgebildet ist.

**7.** Messzelle (3) nach einem der Ansprüche 1 bis 5,
bei der der Transistor (V) als ein in Emitter-Schaltung betriebener Bipolartransistor ausgebildet ist.

**8.** Messzelle (3) nach einem der Ansprüche 1 bis 5,
bei der der Transistor (V) in einer Darlington-Schaltung verbundene Subtransistoren enthält.

**9.** Messzelle (3) nach einem der Ansprüche 5 bis 8,
bei der das Lastelement ($R_1$) als ohmscher Widerstand ausgebildet ist.

**10.** Messzelle (3) nach einem der Ansprüche 5 bis 8,
bei der das Lastelement ($R_1$) als Transistor ausgebildet ist.

**11.** Messzelle (3) nach einem der Ansprüche 5 bis 10,
bei der das Lastelement ($R_1$) in seiner Last einstellbar ist.

**12.** Messzelle (3) nach einem der Ansprüche 5 bis 11,
bei der das Lastelement ($R_1$) auf dem Substrat (2) angeordnet ist.

**13.** Messzelle (3) nach einem der Ansprüche 5 bis 11,
bei der das Lastelement ($R_1$) außerhalb des Substrats (2) angeordnet ist.

**14.** Messzelle (3) nach einem der Ansprüche 4 bis 13,
bei der der Schalter ($P_A$) als Transistor ausgebildet ist.

**15.** Messzelle (3) nach einem der Ansprüche 4 bis 14,
bei der der Schalter ($P_A$) auf dem Substrat (2) angeordnet ist.

**16.** Messzelle (3) nach einem der Ansprüche 4 bis 15,
bei der der Steueranschluss des Transistors (V) über ein weiteres Lastelement ($R_2$) mit Drain beziehungsweise dem Kollektor des Transistors (V) verbunden ist.

**17.** Messzelle (3) nach Anspruch 16,
bei der das weitere Lastelement ($R_2$) auf dem Substrat (2) angeordnet ist.

**18.** Messzelle (3) nach einem der vorhergehenden Ansprüche, bei der das Substrat (2) zumindest Anschlüsse aufweist für:

• ein erstes Versorgungspotential,
• ein weiteres Versorgungspotential,

• ein elektrisches Ausgangssignal.

**19.** Messzelle (3) nach einem der Ansprüche 5 bis 18,
bei der ein weiterer steuerbarer Schalter. ($P_M$) mit Drain beziehungsweise dem Kollektor des Transistors (V) verbunden ist.

**20.** Messzelle (3) nach Anspruch 19,
bei der der weitere steuerbare Schalter ($P_M$) zwischen Drain beziehungsweise dem Kollektor des Transistors (V) und dem Lastelement ($R_1$) angeordnet ist.

**21.** Messzelle (3) nach Anspruch 19 oder Anspruch 20,
bei der der weitere steuerbare Schalter ($P_M$) auf dem Substrat (2) angeordnet ist.

**22.** Messzelle (3) nach einem der vorhergehenden Ansprüche,
bei der das Wandlerelement (A) einen Feldeffekttransistor mit Gate, Source und Drain enthält, der derart ausgebildet ist, dass die Probe (4) im Messbetrieb über dem Gate angeordnet und ihr elektrisches Potential mit einem Kanalstrom des Feldeffekttransistors zwischen Source und Drain gekoppelt ist.

**23.** Messzelle (3) nach einem der vorhergehenden Ansprüche,
bei der das Wandlerelement (A) eine pH-sensitive Schicht enthält.

**24.** Messzelle (3) nach einem der vorhergehenden Ansprüche,
bei der das Wandlerelement (A) eine stoffselektive Membran enthält.

**25.** Messfeld (1),
enthaltend eine Vielzahl von Messzellen (3) nach einem der vorhergehenden Ansprüche auf einem gemeinsamen Substrat (2).

**26.** Messfeld (1) nach Anspruch 25,
bei dem die Messzellen (3) in Reihen und Spalten auf dem Substrat (2) angeordnet sind.

**27.** Messfeld (1) nach Anspruch 26,
bei dem das Substrat (2) eine gemeinsame Source-Verbindungsleitung (SL) aufweist zum Verbinden von Source- bzw. Emitteranschlüssen entweder einer Zellenreihe oder einer Zellenspalte.

**28.** Messfeld (1) nach Anspruch 27,

• bei dem je Zellenreihe beziehungsweise Zellenspalte eine solche gemeinsame Source-Verbindungsleitung (SL) vorgesehen ist, und
• bei dem das Substrat (2) einen Anschluss für jede Source-Verbindungsleitung (SL) aufweist.

**29.** Messfeld (1) nach Anspruch 27 oder Anspruch 28,
bei dem das Substrat (2) eine gemeinsame Steuerleitung (STL) zum Steuern von Schaltern entweder einer Zellenspalte oder einer Zellenreihe aufweist.

**30.** Messfeld (1) nach Anspruch 29,

• bei dem je Zellenspalte beziehungsweise Zellenreihe eine solche gemeinsame Steuerleitung (STL) vorgesehen ist, und
• bei der das Substrat (2) einen Anschluss für jede Steuerleitung (STL) aufweist.

**31.** Messfeld nach einem der Ansprüche 27 bis 30,
bei dem das Substrat (2) eine gemeinsame weitere Steuerleitung (WSL) zum Steuern von weiteren Schaltern entweder einer Zellenreihe oder einer Zellenspalte aufweist.

**32.** Messfeld (1) nach Anspruch 31,

• bei dem je Zellenreihe beziehungsweise Zellenspalte eine solche gemeinsame weitere Steuerleitung (WSL) vorgesehen ist, und
• bei der das Substrat (2) einen Anschluss für jede Weitere Steuerleitung (WSL) aufweist.

33. Messfeld (1) nach einem der Ansprüche 27 bis 32 in Verbindung mit Messzellen (3) nach Anspruch 19, bei dem das Substrat eine gemeinsame Ausgangsleitung (AL) zum Verbinden von weiteren Schaltern ($P_M$) entweder einer Zellenspalte oder einer Zellenreihe aufweist.

34. Messfeld (1) nach Anspruch 33,

   • bei dem je Zellenspalte beziehungsweise Zellenreihe eine solche gemeinsame Ausgangsleitung (AL) vorgesehen ist, und
   • bei der das Substrat (2) einen Anschluss für jede Ausgangsleitung (AL) aufweist.

35. Messfeld (1) nach einem der Ansprüche 27 bis 34, bei dem das Substrat (2) eine gemeinsame lastseitige Versorgungsleitung (LVL) enthält zum Verbinden von Lastelementen (R) entweder einer Zellenspalte oder einer Zellenreihe.

36. Messfeld (1) nach Anspruch 35,

   • bei dem je Zellenspalte beziehungsweise Zellenreihe eine solche gemeinsame lastseitige Versorgungsleitung (LVL) vorgesehen ist, und
   • bei der das Substrat (2).einen Anschluss für jede weitere lastseitige Versorgungsleitung (LVL) aufweist.

37. Messfeld (1) nach einem der Ansprüche 27 bis 34 in Verbindung mit Messzellen (3) nach Anspruch 20, bei dem das Substrat (2) eine gemeinsame Verbindungsleitung (VL) enthält zum Verbinden von weiteren steuerbaren Schaltern ($P_S$) entweder einer Zellenspalte oder einer Zellenreihe.

38. Messfeld (1) nach Anspruch 37,

   • bei dem je Zellenspalte beziehungsweise Zellenreihe eine solche gemeinsame Verbindungsleitung (VL) vorgesehen ist,
   • bei der das Substrat (2) einen ersten Anschluss für jede Verbindungsleitung (VL) aufweist,
   • bei dem ein Lastelement je Zellenspalte beziehungsweise je Zellenreihe außerhalb des Substrats (2) angeordnet ist,
   • bei dem jedes Lastelement mit einem Anschluss für eine Verbindungsleitung (VL) verbunden ist, und
   • bei dem das Substrat (2) einen weiteren Anschluss zum Abgreifen eines elektrischen Ausgangssignals für jede Verbindungsleitung (VL) aufweist.

39. Messfeld (1) nach einem der Ansprüche 27 bis 34,

   • bei dem jede Messzelle (3) eine Diode ($D_S$) enthält, die mit Drain beziehungsweise dem Kollektor des Transistors (V) verbunden ist, und
   • bei dem das Substrat (2) eine gemeinsame Verbindungsleitung (VL) enthält zum Verbinden aller Dioden ($D_S$) entweder einer Zellenspalte oder einer Zellenreihe.

40. Messfeld (1) nach Anspruch 39,

   • bei dem je Zellenspalte beziehungsweise Zellenreihe eine solche gemeinsame Verbindungsleitung (VL) vorgesehen ist,
   • bei dem das Substrat (2) einen ersten Anschluss für jede Verbindungsleitung (VL) aufweist,
   • bei dem ein Lastelement je Zellenspalte beziehungsweise je Zellenreihe außerhalb des Substrats (2) angeordnet ist,
   • bei dem jedes Lastelement mit einem Anschluss für eine Verbindungsleitung (VL) verbunden ist, und
   • bei dem das Substrat (2) einen weiteren Anschluss zum Abgreifen eines elektrischen Ausgangssignals für jede Verbindungsleitung (VL) aufweist.

41. Messfeld (1) nach einem der Ansprüche 25 bis 30, bei dem die Wandlerelemente (A) mindestens zweier unterschiedlicher Messzellen (3) zur Erkennung unterschiedlicher Parameter oder Substanzen der Probe (4) ausgebildet sind.

**42.** Messfeld (1) nach einem der Ansprüche 25 bis 41,
bei der die Messzellen (3) den Boden einer Wanne (6) bilden, die im Messbetrieb die Probe (4) aufnimmt.

**43.** Messfeld (1) nach einem der Ansprüche 25 bis 42,
mit einem Kanal (5) über den Messzellen (3) zum Aufnehmen der Probe (4) im Messbetrieb.

**44.** Verfahren zum Einstellen eines Arbeitspunktes einer Verstärkerschaltung (B) in einer Messzelle (3), welche eingerichtet ist zum Aufnehmen eines elektrischen Potentials eines sich auf der Messzelle (3) befindenden Analyten,

• wobei die Messzelle ein Wandlerelement (A) aufweist, welches eingerichtet ist zum Umsetzen einer Messgröße in ein elektrisches Potential, wobei das Wandlerelement (A) eine Elektrode aufweist,
• wobei die Messzelle (3) ferner eine Verstärkerschaltung (B) aufweist, welche über eine Entkopplungskapazität ($C_E$) mit dem Wandlerelement (A) verbunden ist, wobei die Verstärkerschaltung (B) eine Eingangsstufe aufweist, wobei die Eingangsstufe der Verstärkerschaltung (B) einen Feldeffekttransistor (V) oder einen Bipolartransistor (V) enthält,
• wobei die Elektrode zumindest mittelbar mit einem Steueranschluss des Feldeffekttransistors (V) bzw. des Bipolartransistors (V) der Eingangsstufe der Verstärkerschaltung (B) verbunden ist, und
• wobei die Elektrode und die Verstärkerschaltung (B) auf einem gemeinsamen Substrat integriert angeordnet sind,

wobei gemäß dem Verfahren der Arbeitspunkt der Verstärkerschaltung (B) eingestellt.wird über die Einprägung einer geeigneten Spannung oder eines geeigneten Stromes an dem Steueranschluss des Feldeffekttransistors (V) bzw. des Bipolartransistors (V) der Eingangsstufe der Verstärkerschaltung (B), wobei die Verstärkerschaltung (B) eine Rückkopplung aufweist, über welche die Einprägung der Spannung bzw. des Stromes an dem Steueranschluss des Feldeffekttransistors (V) bzw. des Bipolartransistors (V) erfolgt, derart, dass Parameterschwankungen der Verstärkerschaltung (B) automatisch kompensiert werden.

**45.** Verfahren nach Anspruch 44,

• wobei die Rückkopplung der Verstärkerschaltung (B) als gesteuerter Schalter ($P_A$) eingerichtet ist;
• bei dem in einer Messbetriebsphase zum Messen des Potentials der Probe (4) der gesteuerte Schalter ($P_A$) geöffnet ist, und
• bei dem in einer Einstellbetriebsphase zum Einstellen des Arbeitspunktes der Verstärkerschaltung (B) der gesteuerte Schalter ($P_A$) geschlossen wird.

**46.** Verwendung einer Messzelle (3) nach einem der Ansprüche 1 bis 24 zum Beaufschlagen einer Probe (4) mit einem elektrischen Potential durch Anlegen eines elektrischen Signals an die Verstärkerschaltung (B).

**47.** Verwendung eines Messfeldes (1) nach einem der Ansprüche 25 bis 43 zum ortsaufgelösten Beaufschlagen einer Probe (4) mit elektrischen Potentialen durch Anlegen von elektrischen Signalen an die Verstärkerschaltung (B) der einzelnen Messzellen (3).

**Claims**

**1.** A measuring cell (3) for recording an electrical potential of an analyte situated on the measuring cell (3) having

• a converter element (A), which is arranged for converting of a measuring quantity into an electrical potential, wherein the converter element (A) comprises an electrode;
• an amplifier circuit (B), which is connected to the converter element (A) through a decoupling capacity ($C_E$), wherein the amplifier circuit (B) has an input stage, wherein die input stage of the amplifier circuit (B) has a field-effect transistor (V) or a bipolar transistor (V), and wherein the electrode is at least indirectly connected to a control terminal of the field-effect transistor (V) or of the bipolar transistor (V) of the input stage of the amplifier circuit (B), and
• an operating point setting unit ($R_1$, $R_2$, $P_A$, $P_M$) for setting of a suitable operating point of the amplifier circuit (B), wherein the operating point setting unit is arranged such that the setting of a suitable operating point of the amplifier circuit (B) is effected by impressing of a suitable voltage or a suitable current at the control terminal of the field-effect transistor (V) or of the bipolar transistor (V) of the input stage of the amplifier circuit (B), and wherein the amplifier circuit (B) comprises a feedback, by means of which the impressing of the voltage or the current at the control terminal of the field effect transistor (V) or the bipolar transistor (V) is effected in such a manner that parameter variations of the amplifier circuit (B) are automatically compensated; and
• in which the electrode and the amplifier circuit (B) are arranged integrated on a common substrate (2).

2. The measuring cell (3) as claimed in claim 1,
in which the amplifier circuit (B) is configured in single-stage fashion.

3. The measuring cell (B) as claimed in claim 1,
in which the amplifier circuit (B) is configured in multistage fashion.

4. The measuring cell as claimed in one of claims 1 to 3,
in which the feedback is set up as

• a controlled switch ($P_A$),
• a low-pass, or
• a unit for externally storing an operating voltage.

5. The measuring cell (3) as claimed in one of claims 1 to 4,

• in which at least one load element ($R_1$) is provided for setting the operating point of the field-effect transistor (V) or of the bipolar transistor (V),
• in which the drain or the collector of the transistor (V) is connected to a first supply potential via the load element ($R_1$), and
• in which the control terminal of the field-effect transistor (V) or of the bipolar transistor (V) is connected to the drain or the collector of the transistor (V) via a controllable switch ($P_A$).

6. The measuring cell (3) as claimed in one of claims 1 to 5,
in which the transistor (V) is designed as a field-effect transistor operated in source connection.

7. The measuring cell (3) as claimed in one of claims 1 to 5,
in which the transistor (V) is designed as a bipolar transistor operated in an emitter connection.

8. The measuring cell (3) as claimed in one of claims 1 to 5,
in which the transistor (V) contains sub-transistors connected in a Darlington circuit.

9. The measuring cell (3) as claimed in one of claims 5 to 8,
in which the load element ($R_1$) is designed as an ohmic resistor.

10. The measuring cell (3) as claimed in one of claims 5 to 8,
in which the load element ($R_1$) is designed as a transistor.

11. The measuring cell (3) as claimed in one of claims 5 to 10,
in which the load of the load element ($R_1$) is adjustable.

12. The measuring cell (3) as claimed in one of claims 5 to 11,
in which the load element ($R_1$) is arranged on the substrate (2).

13. The measuring cell (3) as claimed in one of claims 5 to 11,
in which the load element ($R_1$) is arranged outside the substrate (2).

14. The measuring cell (3) as claimed in one of claims 4 to 13,
in which the switch ($P_A$) is designed as a transistor.

15. The measuring cell (3) as claimed in one of claims 4 to 14,
in which the switch ($P_A$) is arranged on the substrate (2).

16. The measuring cell (3) as claimed in one of claims 4 to 15,

in which the control terminal of the transistor (V) is connected to the drain or the collector of the transistor (V) via a further load element ($R_2$).

17. The measuring cell (3) as claimed in claim 16,
in which the further load element ($R_2$) is arranged on the substrate (2).

18. The measuring cell (3) as claimed in one of the preceding claims,
in which the substrate (2) at least has terminals for:

　　　　• a first supply potential,
　　　　• a further supply potential,
　　　　• an electrical output signal.

19. The measuring cell (3) as claimed in one of claims 5 to 18,
in which a further controllable switch ($P_M$) is connected to the drain or the collector of the transistor (V).

20. The measuring cell (3) as claimed in claim 19,
in which the further controllable switch ($P_M$) is arranged between the drain or the collector of the transistor (V) and the load element ($R_1$).

21. The measuring cell (3) as claimed in claim 19 or claim 20,
in which the further controllable switch ($P_M$) is arranged on the substrate (2).

22. The measuring cell (3) as claimed in one of the preceding claims,
in which the converter element (A) contains a field-effect transistor having gate, source and drain, which is designed in such a way that the sample (4) is arranged above the gate during measuring operation and its electrical potential is coupled to a channel current of the field-effect transistor between source and drain.

23. The measuring cell (3) as claimed in one of the preceding claims,
in which the converter element (A) contains a pH-sensitive layer.

24. The measuring cell (3) as claimed in one of the preceding claims,
in which the converter element (A) contains a substance-selective membrane.

25. A measuring array (1),
containing a multiplicity of measuring cells (3) as claimed in one of the preceding claims on a common substrate (2).

26. The measuring array (1) as claimed in claim 25,
in which the measuring cells (3) are arranged in rows and columns on the substrate (2).

27. The measuring array (1) as claimed in claim 26,
in which the substrate (2) has a common source connecting line (SL) for connecting source or emitter terminals either of a cell row or of a cell column.

28. The measuring array (1) as claimed in claim 27,

　　　　• in which such a common source connecting line (SL) is provided for each cell row or cell column, and
　　　　• in which the substrate (2) has a terminal for each source connecting line (SL).

29. The measuring array (1) as claimed in claim 27 or claim 28,
in which the substrate (2) has a common control line (STL) for controlling switches either of a cell column or of a cell row.

30. The measuring array (1) as claimed in claim 29,

　　　　• in which such a common control line (STL) is provided for each cell column or cell row, and
　　　　• in which the substrate (2) has a terminal for each control line (STL).

**31.** The measuring array (1) as claimed in one of claims 27 to 30,
in which the substrate (2) has a common further control line (WSL) for controlling further switches either of a cell row or of a cell column.

**32.** The measuring array (1) as claimed in claim 31,

- in which such a common further control line (WSL) is provided for each cell row or cell column, and
- in which the substrate (2) has a terminal for each further control line (WSL).

**33.** The measuring array (1) as claimed in one of claims 27 to 32 in conjunction with measuring cells (3) as claimed in claim 19,
in which the substrate has a common output line (AL) for connecting further switches ($P_M$) either of a cell column or of a cell row.

**34.** The measuring array (1) as claimed in claim 33,

- in which such a common output line (AL) is provided for each cell column or cell row, and
- in which the substrate (2) has a terminal for each output line (AL).

**35.** The measuring array (1) as claimed in one of claims 27 to 34,
in which the substrate (2) contains a common load-side supply line (LVL) for connecting load elements (R) either of a cell column or of a cell row.

**36.** The measuring array (1) as claimed in claim 35,

- in which such a common load-side supply line (LVL) is provided for each cell column or cell row, and
- in which the substrate (2) has a terminal for each further load-side supply line (LVL).

**37.** The measuring array (1) as claimed in one of claims 27 to 34 in conjunction with measuring cells (3) as claimed in claim 20,
in which the substrate (2) contains a common connecting line (VL) for connecting further controllable switches ($P_S$) either of a cell column or of a cell row.

**38.** The measuring array (1) as claimed in claim 37,

- in which such a common connecting line (VL) is provided for each cell column or cell row,
- in which the substrate (2) has a first terminal for each connecting line (VL),
- in which a load element is arranged outside the substrate (2) for each cell column or for each cell row,
- in which each load element is connected to a terminal for a connecting line (VL), and
- in which the substrate (2) has a further terminal for tapping off an electrical output signal for each connecting line (VL).

**39.** The measuring array (1) as claimed in one of claims 27 to 34,

- in which each measuring cell (3) contains a diode ($D_S$) connected to the drain or the collector of the transistor (V), and
- in which the substrate (2) contains a common connecting line (VL) for connecting all the diodes ($D_S$) either of a cell column or of a cell row.

**40.** The measuring array (1) as claimed in claim 39,

- in which such a common connecting line (VL) is provided for each cell column or cell row,
- in which the substrate (2) has a first terminal for each connecting line (VL),
- in which a load element is arranged outside the substrate (2) for each cell column or for each cell row,
- in which each load element is connected to a terminal for a connecting line (VL), and
- in which the substrate (2) has a further terminal for tapping off an electrical output signal for each connecting line (VL).

**41.** The measuring array (1) as claimed in one of claims 25 to 30,
in which the converter elements (A) of at least two different measuring cells (3) are designed for detecting different parameters or substances of the sample (4).

**42.** The measuring array (1) as claimed in one of claims 25 to 41,
in which the measuring cells (3) form the bottom of a well (6) which receives the sample (4) during measuring operation.

**43.** The measuring array (1) as claimed in one of claims 25 to 42,
having a channel (5) above the measuring cells (3) for receiving the sample (4) during measuring operation.

**44.** Method for setting the operating point of an amplifier circuit (B) in a measuring cell (3), which is set up for receiving an electrical potential of an analyte situated on the measuring cell (3),

• wherein the measuring cell comprises a converter element (A), which is arranged for converting of a measuring quantity into an electrical potential, wherein the converter element (A) comprises an electrode,
• wherein the measuring cell (3) further comprises an amplifier circuit (B), which is connected to the converter element (A) through a decoupling capacity ($C_E$), wherein the amplifier circuit (B) has an input stage, wherein the input stage of the amplifier circuit (B) has a field-effect transistor (V) or a bipolar transistor (V),
• wherein the electrode is at least indirectly connected to a control terminal of the field-effect transistor (V) or the bipolar transistor (V) of the input stage of the amplifier circuit (B), and
• wherein the electrode and the amplifier circuit (B) are arranged integrated on a common substrate

wherein according to the method the operating point of the amplifier circuit (B) is set by impressing of a suitable voltage or a suitable current at the control terminal of the field-effect transistor (V) or the bipolar transistor (V) of the input stage of the amplifier circuit (B), wherein the amplifier circuit (B) comprises a feedback, by means of which the impressing of the voltage or the current at the control terminal of the field effect transistor (V) or the bipolar transistor (V) is effected in such a manner that parameter variations of the amplifier circuit (B) are automatically compensated.

**45.** The method as claimed in claim 44,

• wherein the feedback of the amplifier circuit (B) is arranged as controlled switch ($P_A$);
• in which the controlled switch ($P_A$) is opened in a measurement operating phase for measuring the potentials of the sample (4), and
• in which the controlled switch ($P_A$) is closed in a setting operation phase for setting the operating point of the amplifier circuit (B).

**46.** The use of a measuring cell (3) as claimed in one of claims 1 to 24 for supplying an electrical potential to a sample (4) by the application of an electrical signal to the amplifier circuit (B).

**47.** The use of a measuring array (1) as claimed in one of claims 25 to 43 for supplying electrical potentials to a sample (4) in a spatially resolved manner by the application of electrical signals to the amplifier circuit (B) of the individual measuring cells (3).

**Revendications**

**1.** Cellule (3) de mesure pour l'enregistrement d'un potentiel électrique d'un analyte se trouvant sur la cellule (3) de mesure,

• ayant un élément (A) de transducteur, qui est conçu pour transformer une grandeur de mesure en un potentiel électrique, l'élément (A) de transducteur ayant une électrode ;
• ayant un circuit (B) d'amplificateur, qui est relié à l'élément (A) de transducteur par une capacité ($C_E$) de découplage, le circuit (B) d'amplificateur ayant un étage d'entrée, l'étage d'entrée du circuit (B) d'amplificateur comportant un transistor (V) à effet de champ ou un transistor (V) bipolaire et l'électrode étant reliée au moins indirectement à une borne de commande du transistor (V) à effet de champ ou du transistor (V) bipolaire de l'étage d'entrée du circuit (B) d'amplificateur ; et

• ayant une unité (R$_1$, R$_2$, P$_A$, P$_M$) de réglage du point de travail pour régler un point de travail approprié du circuit (B) d'amplificateur, l'unité de réglage du point de travail étant telle que le réglage d'un point de travail approprié du circuit (B) d'amplificateur s'effectue par l'application d'une tension appropriée ou d'un courant approprié à la borne de commande du transistor (V) à effet de champ ou du transistor (V) bipolaire de l'étage d'entrée du circuit (B) d'amplificateur et le circuit (B) d'amplificateur comportant une réaction par laquelle l'application de la tension ou du courant à la borne de commande du transistor (V) à effet de champ ou du transistor (V) bipolaire s'effectue de manière à compenser automatiquement des fluctuations des paramètres du circuit (B) d'amplificateur ; et

• dans laquelle l'électrode et le circuit (B) d'amplificateur sont disposés de façon intégrée sur un substrat (2) commun.

2. Cellule (3) de mesure suivant la revendication 1,
dans laquelle le circuit (B) d'amplificateur est à un étage.

3. Cellule (3) de mesure suivant la revendication 1,
dans laquelle le circuit (B) d'amplificateur est à plusieurs étages.

4. Cellule (3) de mesure suivant l'une des revendications 1 à 3,
dans laquelle la réaction est sous la forme

• d'un interrupteur (P$_A$) commandé,
• d'un passe-bas ou
• d'une unité d'emmagasinage extérieure d'une tension de fonctionnement.

5. Cellule (3) de mesure suivant l'une des revendications 1 à 4,

• dans laquelle pour régler le point de travail du transistor (V) à effet de champ ou du transistor (B) bipolaire il est prévu au moins un élément (R$_1$) de charge,
• dans laquelle le drain ou le collecteur de transistor (V) est relié à un premier potentiel d'alimentation par l'élément (R$_1$) de charge et
• dans laquelle la borne de commande du transistor (V) à effet de champ ou du transistor (V) bipolaire est reliée au drain ou au collecteur du transistor (V) par un interrupteur (P$_A$) qui peut être commandé.

6. Cellule (3) de mesure suivant l'une des revendications 1 à 5,
dans laquelle le transistor (V) est constitué sous la forme d'un transistor à effet de champ fonctionnant en circuit source.

7. Cellule (3) de mesure suivant l'une des revendications 1 à 5,
dans laquelle le transistor (V) est constitué sous la forme d'un transistor bipolaire fonctionnant en circuit émetteur.

8. Cellule (3) de mesure suivant l'une des revendications 1 à 5,
dans laquelle le transistor (V) comporte des sous-transistors montés en circuit Darlington.

9. Cellule (3) de mesure suivant l'une des revendications 5 à 8,
dans laquelle l'élément (R$_1$) de charge est constitué sous la forme d'une résistance ohmique.

10. Cellule (3) de mesure suivant l'une des revendications 5 à 8,
dans laquelle l'élément (R$_1$) de charge est constitué sous la forme d'un transistor.

11. Cellule (3) de mesure suivant l'une des revendications 5 à 10,
dans laquelle la charge de l'élément (R$_1$) de charge est réglable.

12. Cellule (3) de mesure suivant l'une des revendications 5 à 11,
dans laquelle l'élément (R$_1$) de charge est disposé sur le substrat (2).

13. Cellule (3) de mesure suivant l'une des revendications 5 à 11,
dans laquelle l'élément (R$_1$) de charge est disposé à l'extérieur du substrat (2).

**14.** Cellule (3) de mesure suivant l'une des revendications 4 à 13,
dans laquelle l'interrupteur (P$_A$) est constitué sous la forme d'un transistor.

**15.** Cellule (3) de mesure suivant l'une des revendications 4 à 14,
dans laquelle l'interrupteur (P$_A$) est disposé sur le substrat (2).

**16.** Cellule (3) de mesure suivant l'une des revendications 4 à 15,
dans laquelle la borne de commande du transistor (V) est reliée au drain ou au collecteur du transistor (V) par un autre élément (R$_2$) de charge.

**17.** Cellule (3) de mesure suivant la revendication 16,
dans laquelle l'autre élément (R$_2$) de charge est disposé sur le substrat (2).

**18.** Cellule (3) de mesure suivant l'une des revendications précédentes,
dans laquelle le substrat (2) a au moins des bornes pour :

  • un premier potentiel d'alimentation,
  • un autre potentiel d'alimentation,
  • un signal électrique de sortie.

**19.** Cellule (3) de mesure suivant l'une des revendications 5 à 18,
dans laquelle un autre interrupteur (P$_M$) pouvant être commandé est relié au drain ou au collecteur du transistor (V).

**20.** Cellule (3) de mesure suivant la revendication 19,
dans laquelle l'autre interrupteur (P$_M$) pouvant être commandé est disposé entre le drain ou le collecteur du transistor (V) et l'élément (R$_1$) de charge.

**21.** Cellule (3) de mesure suivant la revendication 19 ou la revendication 20,
dans laquelle l'autre interrupteur (P$_M$) pouvant être commandé est disposé sur le substrat (2).

**22.** Cellule (3) de mesure suivant l'une des revendications précédentes,
dans laquelle l'élément (A) de transducteur comporte un transistor à effet de champ ayant une grille, une source et un drain, qui est telle que l'échantillon (4) est disposé en fonctionnement de mesure sur la grille et que son potentiel électrique est couplé à un courant de canal du transistor à effet de champ entre la source et le drain.

**23.** Cellule (3) de mesure suivant l'une des revendications précédentes,
dans laquelle l'élément (A) de transducteur comporte une couche sensible au pH.

**24.** Cellule (3) de mesure suivant l'une des revendications précédentes,
dans laquelle l'élément (A) de transducteur comporte une membrane sélective aux substances.

**25.** Champ (1) de mesure contenant une pluralité de cellules (3) de mesure suivant l'une des revendications précédentes sur un substrat (2) commun.

**26.** Champ (1) de mesure suivant la revendication 25,
dans lequel les cellules (3) de mesure sont disposées en lignes et en colonnes sur le substrat (2).

**27.** Champ (1) de mesure suivant la revendication 26,
dans lequel le substrat (2) a une ligne (SL) de liaison source commune pour la liaison de borne, de source ou d'émetteur, soit d'une ligne de cellules, soit d'une colonne de cellules.

**28.** Champ (1) de mesure suivant la revendication 27,

  • dans lequel il est prévu une telle ligne (DL) de liaison de source commune pour, respectivement, une ligne de cellules ou une colonne de cellules, et
  • dans lequel le substrat (2) a une borne pour chaque ligne (SL) de liaison de source.

**29.** Champ (1) de mesure suivant la revendication 27 ou la revendication 28,

dans lequel le substrat (2) a une ligne (STL) de commande commune pour commander des interrupteurs d'une colonne de cellules ou d'une ligne de cellules.

**30.** Champ (1) de mesure suivant la revendication 29,

• dans lequel une ligne (STL) de commande commune de ce genre est prévue pour chaque colonne de cellules ou ligne de cellules et
• dans lequel le substrat (2) a une borne pour chaque ligne (STL) de commande.

**31.** Champ (1) de mesure suivant l'une des revendications 27 à 30,
dans lequel le substrat (2) a une autre ligne (WSL) de commande pour commander d'autres interrupteurs d'une ligne de cellules ou d'une colonne de cellules.

**32.** Champ (1) de mesure suivant la revendication 31,

• dans lequel une autre ligne (WSL) de commande commune de ce genre est prévue pour chaque ligne de cellules ou colonne de cellules et
• dans lequel le substrat (2) a une borne pour chaque autre ligne (WSL) de commande.

**33.** Champ (1) de mesure suivant l'une des revendications 27 à 32 en liaison avec des cellules (3) de mesure suivant la revendication 19,
dans lequel le substrat a une ligne (AL) commune de sortie pour relier d'autres interrupteurs ($P_M$) d'une colonne de cellules ou d'une ligne de cellules.

**34.** Champ (1) de mesure suivant la revendication 33,

• dans lequel il est prévu une ligne (AL) commune de sortie de ce genre pour chaque colonne de cellules ou pour chaque ligne de cellules et
• dans lequel le substrat (2) a une borne pour chaque ligne (AL) de sortie.

**35.** Champ (1) de mesure suivant l'une des revendications 27 à 34,
dans lequel le substrat (2) comporte une ligne (LVL) d'alimentation commune du côté de la charge pour relier des éléments (R) de charge d'une colonne cellules ou d'une ligne de cellules.

**36.** Champ (1) de mesure suivant la revendication 35,

• dans lequel il est prévu une ligne (LVL) d'alimentation commune du côté de la charge de ce genre pour chaque colonne de cellules ou chaque ligne de cellules et
• dans lequel le substrat (2) a une borne pour chaque autre ligne (LVL) d'alimentation du côté de la charge.

**37.** Champ (1) de mesure suivant l'une des revendications 27 à 34 en liaison avec des cellules (3) de mesure suivant la revendication 20,
dans lequel le substrat (2) comporte une ligne (VL) commune de liaison pour relier d'autres interrupteurs ($P_S$) pouvant être commandés d'une colonne de cellules ou d'une ligne de cellules.

**38.** Champ (1) de mesure suivant la revendication 37,

• dans lequel une ligne (VL) de liaison commune de ce genre est prévue pour chaque colonne de cellules ou chaque ligne de cellules,
• dans lequel le substrat (2) a une première borne pour chaque ligne (VL) de liaison,
• dans lequel un élément de charge est disposé pour chaque colonne de cellules ou ligne de cellules à l'extérieur du substrat (2),
• dans lequel chaque élément de charge est relié à une borne pour une ligne (VL) de liaison et
• dans lequel le substrat (2) a une autre borne de prélèvement d'un signal électrique de sortie pour chaque ligne (VL) de liaison.

**39.** Champ (1) de mesure suivant l'une des revendications 27 à 34,

• dans lequel chaque cellule (3) de mesure comporte une diode ($D_S$) qui est reliée au drain ou au collecteur du transistor (V) et
• dans lequel le substrat (2) comporte une ligne (VL) de liaison commune pour relier toutes les diodes ($D_S$) d'une colonne de cellules ou d 'une ligne de cellules.

**40.** Champ (1) de mesure suivant la revendication 39,

• dans lequel une ligne (VL) de liaison commune de ce genre est prévue pour chaque colonne de cellules ou chaque rangée de cellules,
• dans lequel le substrat (2) a une première borne pour chaque ligne (VL) de liaison,
• dans lequel un élément de charge pour chaque colonne de cellules ou chaque ligne de cellules est disposé à l'extérieur du substrat (2),
• dans lequel chaque élément de charge est relié à une borne pour une ligne (VL) de liaison et
• dans lequel le substrat (2) a une autre borne de prélèvement d'un signal électrique de sortie pour chaque ligne (VL) de liaison.

**41.** Champ (1) de mesure suivant l'une des revendications 25 à 30,
dans lequel les éléments (A) de transducteur d'au moins deux cellules (3) de mesure différentes sont constitués pour détecte des paramètres ou des substances différents de l 'échantillon (4).

**42.** Champ (1) de mesure suivant l'une des revendications 25 à 41,
dans lequel les cellules (3) de mesure forment le fond d'une cuvette (6) qui, en fonctionnement de mesure, reçoit l'échantillon (4).

**43.** Champ (1) de mesure suivant l'une des revendications 25 à 42,
comprenant un canal (5) au-dessus des cellules (3) de mesure pour recevoir l'échantillon (4) en fonctionnement de mesure.

**44.** Procédé de réglage d'un point de travail d'un circuit (B) d'amplificateur dans une cellule (3) de mesure, qui est conçu pour enregistrer un potentiel électrique d'un analyte se trouvant sur la cellule (3) de mesure,

• dans lequel la cellule de mesure a un élément (A) de transducteur conçu pour transformer une grandeur de mesure en un potentiel électrique, l'élément (A) de transducteur ayant une électrode,
• dans lequel la cellule (3) de mesure a, en outre, un circuit (B) d'amplificateur, qui est relié à l'élément (A) de transducteur par une capacité ($C_E$) de découplage, le circuit (B) d'amplificateur ayant un étage d'entrée, l'étage d'entrée du circuit (B) d'amplificateur comportant un transistor (V) à effet de champ ou un transistor (V) bipolaire,
• dans lequel l'électrode est reliée au moins indirectement à une borne de commande du transistor (V) à effet de champ ou du transistor (V) bipolaire de l 'étage d 'entrée du circuit (B) d'amplificateur et
• dans lequel l'électrode et le circuit (B) d'amplificateur sont disposés de manière intégrée sur un substrat commun,

dans lequel on règle suivant le procédé le point de travail du circuit (B) amplificateur en appliquant une tension appropriée ou un courant approprié à la borne de commande du transistor (V) à effet de champ ou du transistor (V) bipolaire de l'étage d'entrée du circuit (B) d'amplificateur, le circuit (B) d'amplificateur ayant une réaction par laquelle s'effectue l'application de la tension ou du courant à la borne de commande du transistor (V) à effet de champ ou du transistor (V) bipolaire, de manière à compenser automatiquement des fluctuations de paramètre du circuit (B) d'amplificateur.

**45.** Procédé suivant la revendication 44,

• dans lequel la réaction du circuit (B) d'amplificateur est conçue sous la forme d'un interrupteur ($P_A$) commandé ;
• dans lequel dans une phase de fonctionnement de mesure on ouvre l'interrupteur ($P_A$) commandé pour la mesure du potentiel de l'échantillon (4) et
• dans lequel dans une phase de fonctionnement de réglage on ferme l'interrupteur ($P_A$) commandé pour le réglage du point de travail du circuit (B) d'amplificateur.

**46.** Utilisation d'une cellule (3) de mesure suivant l'une des revendications 1 à 24 pour appliquer à un échantillon (4) un potentiel électrique par application d'un signal électrique au circuit (B) d'amplificateur.

47. Utilisation d'un champ (1) mesure suivant l'une des revendications 25 à 43 pour appliquer avec résolution spatiale des potentiels électriques à un échantillon (4) en appliquant des signaux électriques au circuit (B) amplificateur des diverses cellules (3) de mesure.

FIG 1 $3$

$V_{DD}$  $V_{DD}$

$V_{Analyt}$  $C_E$  $R_2$  $R_1$  $V$

Gate  Drain  $V_{Out}$

Source

$R_3$

A  B

FIG 2

$V_{DD}$

B  $R_1$

$V_{Analyt}$  $R_2$  $V$

Gate  Drain  $V_{Out}$

Source

$C_E$

A

FIG 3

$V_{DD}$

B  $R_1$

$V_{Analyt}$  $C_E$  $P_A$  $V$

Gate  Drain  $V_{Out}$

Source

A

# FIG 4

# FIG 5

# FIG 6

# FIG 7

# FIG 8

# FIG 9

## FIG 10

a) $V_{DD}$
$R_1$
Gate | Drain
Source
$V$

b) $V_{SS}$
$R_1$
$V$

c) $V_{DD}$
$R_1$
$V$

d) $V_{SS}$
$R_1$
$V$

e) $V_{DD}$
$R_1$
$V_V$
$V_V$
$V$

## FIG 11

a) $V_{DD}$
$R_1$
Gate | Drain
Source

b)

c)

d)

e)

f)

EP 1 405 071 B1

# FIG 12

# FIG 13

# FIG 14

# FIG 15

# FIG 16

# FIG 17

## FIG 18

FIG 19

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0942259 A1
- US 5309085 A
- US 5965452 A

**In der Beschreibung aufgeführte Nicht-Patentliteratur**

- **K. TAKAHASHI ; S. TAKEUCHI.** Bi-MOSFET Amplifier for Integration with Multimicroelectrode Array for Extracellular Neuronal Recording. *IEICE TRANS. FUNDAMENTALS,* Februar 1994, vol. E77-A (2), 388-393
- **P. FROMHERZ.** Interfacing Neurons and Silicon by Electrical Induction. *Ber. Bunsenges. Phys. Chem.,* 1996, vol. 100 (7), 1093-1102
- **A. STETT et al.** Two-way silicon-neuron interface by electrical induction. *Physical Review E,* Februar 1997, vol. 55 (2), 1779-1782
- **S. VASSANELLI ; P. FROMHERZ.** Transistor records of excitable neurons from rat brain. *Appl. Phys.,* 1998, vol. A66, 459-463
- **W.J. PARAK et al.** The field-effect-addressable potentiometric sensor/stimulator (FAPS) - a new concept for a surface potential sensor and stimulator with spatial resolution. *Sensors and Actuators,* 1999, vol. B-58, 497-504
- **T. C. W. YEOW et al.** Design of a Single Cell of an ISFET Array Chip. *Microelectronics: Technology today for the future,* 1995, 62-67
- **T. C. W. YEOW et al.** A very large integrated pH-ISFET sensor array chip compatible with standard CMOS process. *Sensors an Actuators B,* 1997, vol. 44, 434-440
- **T. C. W. YEOW et al.** Thick-Film Thermistor Array Using a Novel Threshold Conversion Concept. *Sensors an Materials,* 1998, vol. 10 (2), 77-91
- **K. VAVELIDIS ; Y. TSIVIDIS.** *IEEE International Symposium on Circuits and Systems,* 1993, 1180-1183
- **O. LIMANN.** Elektronik ohne Ballast: Einführung in die Schaltungstechnik der industriellen Elektronik. Franzis-Verlag, 1987, 35-38
- **K. TSUKADA ; T. MARUIZUMI ; H. MIYAGI.** ISFET Incorporated in a Differential Amplifier. *Electronics and Communications in Japan,* 1988, vol. 71 (3), 82-84